# EUROPÄISCHE PATENTSCHRIFT

(11) **EP 3 313 500 B1**
(45) Veröffentlichungstag und Bekanntmachung des Hinweises auf die Patenterteilung: **31.07.2019**
(21) Anmeldenummer: 16732654.5
(22) Anmeldetag: 28.06.2016
(51) Int. Cl.: A61M 35/00, B05C 17/005

(54) **BEHÄLTER ZUM AUFBEWAHREN UND AUSBRINGEN WENIGSTENS EINER KOMPONENTE SOWIE VERFAHREN HIERFÜR**
CONTAINER FOR STORING AND DISPENSING AT LEAST ONE COMPONENT AND METHOD THEREFOR
RÉCIPIENT POUR CONSERVER ET DISTRIBUER AU MOINS UN COMPOSANT ET PROCÉDÉ ASSOCIÉ

(30) Priorität: 29.06.2015 DE 102015110442
(43) Veröffentlichungstag der Anmeldung: 02.05.2018
(73) Patentinhaber: Kettenbach GmbH & CO. KG, 35713 Eschenburg (DE)
(72) Erfinder: BUBLEWITZ, Alexander, 35745 Herborn (DE); REBER, Jens-Peter, 58540 Meinerzhagen (DE)
(74) Vertreter: Keil & Schaafhausen Patent- und Rechtsanwälte PartGmbB
(86) Internationale Anmeldenummer: PCT/EP2016/065026
(87) Internationale Veröffentlichungsnummer: WO 2017/001412

(56) Entgegenhaltungen:
- WO-A2-2005/021394
- WO-A2-2009/036962
- DE-A1-102007 044 983
- DE-A1-102012 024 408
- US-A1- 2011 198 370

## Beschreibung

Die Erfindung betrifft einen Behälter zum Aufbewahren und Ausbringen wenigstens einer Komponente mit wenigstens einer Kammer, die auf einer ersten (distalen) Seite von einem Stopfen und auf der gegenüberliegenden (proximalen) Seite von einem beweglichen Kolben verschlossen ist, und mit einem auf der ersten Seite befestigten Applikator, wobei der Stopfen relativ zu der Kammer aus einer Lagerposition, in der eine Strömungsverbindung zwischen der Kammer und dem Applikator durch den Stopfen unterbrochen ist, in eine Ausbringposition, in der die Kammer mit dem Applikator in Strömungsverbindung steht, verschiebbar ist.

Derartige Kartuschen zur Verbindung mit einem Mischer als Applikator sind aus der DE 10 2005 041 961 B4 und aus der DE 10 2005 041 962 B4 bekannt.

Aus der EP 1 758 685 B1 ist eine gattungsgemäße Spritze zum Austragen von Mehrkomponentenpasten bekannt. Die Spritze weist zwei konzentrisch angeordnete Kammern auf, welche mit einem Stopfen verschlossen werden. Zum Austragen der Komponenten aus den Kammern wird eine aus mehreren Kolben bestehende Kolbenanordnung verwendet. Wird die Kolbenanordnung zum Austragen der Komponenten verschoben, öffnet der Stopfen durch den erzeugten Innendruck der Komponenten automatisch, indem dieser in Richtung der Austragsöffnung verschoben wird und für jede Komponente ein Austragskanal freigegeben wird. Ein solcher durch den Innendruck der Komponenten bedingter Öffnungsmechanismus für einen Verschlussstopfen wird auch in der WO 2006/132932 A1 und der EP 2 190 592 B1 beschrieben.

Die EP 1 656 215 B2 beschreibt eine Einzeldosisspritze für ein Mehrkomponentenmaterial. An der Kartusche der Spritze ist ein Mischergehäuse mit Mischwendel angeordnet, wobei das Mischergehäuse über einem Bajonettverschluss an der Kartusche festgelegt werden kann. Die Mischwendel kann einstückig mit einem Verschlussstopfen verbunden sein, wobei durch Innendruck der Komponenten die Mischwendel mit dem Verschlussstopfen in Richtung des Auslasses verschoben und so ein Austragen der Komponenten ermöglicht wird. Auch die WO 2005/016783 A1 offenbart einen Verschlussstopfen für zwei Kammern, welcher an einer Mischwendel befestigt ist und zusammen mit dieser durch den Innendruck der Komponenten verschiebbar ist um ein Austragen der Komponenten zu ermöglichen.

Die US 8,616,879 B2 offenbart eine Kapsel zum Aufbewahren und anschließenden Austragen reaktiver oder ätzender Materialien. Zum Öffnen der Kapsel wird ein abreißbarer Vorsatz entfernt. Anschließend kann eine Verschlusskappe oder eine Metallkanüle auf die Kapsel aufgesetzt werden. Eine geeignete Austragspistole für eine solche Kapsel wird in der US 8,602,775 B2 beschrieben.

Die US 5,052,927 beschreibt eine Spritze und eine Einwegkapsel mit Austragskanüle zur Austragung von Dentalmaterial. Die Austragskanüle ist drehbar an der Einwegkapsel angebracht.

Die DE 20 2006 015 457 U1 beschreibt eine Mehrkomponentenkartusche, die einstückig mit einem Mischelement und mit einem über eine Schnappverbindung an der Kartusche festgelegten Mischergehäuse ausgebildet ist. Die auszutragenden Komponenten werden in zwei Kammern gelagert, welche Austragsöffnungen aufweisen, die über einen mit der Innenseite des Anschlussbereiches des Mischers verbundenen Stopfen verschlossen werden. Um die Komponenten auszutragen wird das Mischergehäuse zusammen mit dem Stopfen axial von der Kartusche abgehoben, wodurch die Austragsöffnungen frei gegeben werden.

Auch die US 6,547,101 B offenbart einen Verschlussstopfen für eine Zweikomponentenkartusche, der durch eine axiale Relativbewegung einer Kartusche und eines Applikators aus einer die Kammern der Kartusche verschließenden Position in eine die Kammern freigebende Position verschoben werden kann. Dabei muss der Stopfen gegen die Ausbringrichtung der Komponenten in die Kammern geschoben werden. Einen ähnlichen Öffnungsmechanismus offenbaren auch die EP 1 389 448 A1 und die EP 1 426 017 A2.

Die in der WO 2006/005213 A1 offenbarte Austragsvorrichtung für eine Einmal-Anwendung weist einen auf einer Kartusche festlegbaren Mischer auf, dessen axiale Verschiebung oder dessen Drehung einen Austragkanal der Kartusche freigibt, indem ein Durchlasskanal geöffnet wird oder ein Verschlussstopfen aus dem Austragskanal gezogen wird. Auch eine Kombination beider Bewegungsarten des Mischers zum Öffnen des Austragskanals ist beschrieben. Dabei wird der Mischer auf einer gewindeartigen Schräge der Kartusche geführt, welche eine Drehbewegung des Mischers in eine gleichzeitig stattfindende axiale Verschiebung umsetzt. Diese axiale Verschiebung des Mischers wiederum entfernt einen Verschlussstopfen aus dem Austragskanal der Kartusche, sodass dieser freigegeben wird. Auch die WO 2011/041917 A1 beschreibt ein solches Öffnungssystem für die Austragskanäle, wobei ein Bajonett-Verschluss mit schrägen einen axialen Hub erzeugenden Rampen eingesetzt wird.

In der US 2011/0198370 A1 wird ein Verschlussstopfen beschrieben, der durchgestoßen wird, wenn ein über ein Gewinde mit der Kartusche verbundener Mischer auf der Kartusche festgeschraubt wird.

Die DE 297 09 383 U1 offenbart eine Austrittsvorrichtung mit einem an einer Kartusche angeordneten Auslassstutzen, welcher seitliche Austrittsöffnungen aufweist. Weiter ist eine Verschlusshülse vorgesehen, welche die seitliche Austrittsöffnungen beim Aufsetzen eines Mischers auf die Kartusche freigibt und beim Abnehmen des Mischers von der Kartusche verschließt. Diese Funktion wird durch schräge Eingriffsschlitze und Mitnehmer an der Verschlusshülse erreicht.

Derartige Behälter sind auch als sogenannte preloaded tips (PLT) zum Austragen von Ein- und Mehr-Komponenten-Zahn-Composites bekannt. Diese werden insbesondere für besonders luft- und/oder lichtempfindliche und/oder korrosive und/oder ätzende Komponenten verwendet, welche nur einmal angewendet werden können und/oder sollen. Die Behälter müssen dabei eine hohe Abdichtung und damit eine gute Lagerstabilität der Komponenten erreichen und ein einfaches und sicheres Ausbringen der Komponenten erlauben. Ein Nachteil einiger bekannter Lösungen besteht darin, dass vor der Verwendung des Behälters der Applikator montiert und ggf. ein Verschluss demontiert werden muss. Zudem kann bei einigen bekannten Behältern der die Kammer(n) verschließende Stopfen unbeabsichtigt, bspw. durch Schwankungen des Innendrucks, geöffnet werden.

Die US 2012/0 228 329 A1 A beschreibt eine Austragsvorrichtung mit einer Mehrkomponentenkartusche, einem aufsteckbaren Mischer sowie einer Überwurfmutter zur Festlegung des Mischers auf der Kartusche. Der Mischer weist Verschlusselemente auf, die Austragskanäle der Kartusche verschließen und durch axiale Verschiebung des Mischers in Austragsrichtung freigeben können. Diese axiale Verschiebung des Mischers wird durch ein Verdrehen der Überwurfmutter erreicht.

In der US 2008/0 195 082 A1 wird eine Austragsspritze offenbart, welche konzentrisch angeordnete Kammern aufweist, die im einen Verschlussstopfen verschließbar sind. Der Verschlussstopfen kann in axialer Richtung beim Ausbringen verschoben werden und weist einen Bypasskanal auf, sodass das jeweilige Material aus den Kammern in einen Mischer geführt werden kann.

Die DE 201 06 406 U1 offenbart einen Verschluss für eine Zweikomponentenkartusche mit einem Verschlussstopfen mit zwei Dichtungsbolzen, welche in die Austragsöffnungen der jeweiligen Kammer einsetzbar sind und diese so verschließen. Der Verschlussstopfen kann mit einer Überwurfmutter an der Kartusche festgelegt werden und ist mit der Überwurfmutter über eine lösbare Schnappverbindung verbunden.

Die DE 695 23 561 T2 beschreibt eine Bayonett-Anschlussvorrichtung zum Befestigen eines Zubehörteils, bspw. eines Mischers oder eines Verschlussstopfens, an einer Mehrkomponenten-Kartusche oder Spendervorrichtung.

In der US 2007/0 175 921 A1 ist ein Verschlussstopfen offenbart, welcher zwei Kammern verschließen und nach einer Drehbewegung eines Applikators über den Innendruck beim Ausbringen der Komponenten axial verschoben werden kann und dadurch einen Ausbringkanal freigibt. Dies wird dadurch erreicht, dass am Mischer zwei Vorsprünge derart angeordnet sind, welche in der Verschlussposition eine axiale Verschiebung des Stopfens verhindern. Wird der Mischer um 90° gedreht und eine Austragsposition erreicht, so können die Vorsprünge des Mischers von entsprechend ausgebildeten Aussparungen des Stopfens aufgenommen werden und erlauben so eine axiale Verschiebung des Stopfens und damit eine Freigabe des Strömungskanals, sodass die in den Kammern gelagerten Komponenten ausgetragen werden.

WO 2005/021394 A2 offenbart eine Austragvorrichtung mit einem Verschlussstopfen für eine Kartusche. Der Verschlussstopfen wird über eine Abzugsscheibe derart mit einem Verriegelungsring verbunden, dass eine relative Drehbewegung des Ringes zu der Kartusche den Verschlussstopfen verriegelt oder entriegelt. Nachdem die der Verschlussstopfen entriegelt wurde, kann dieser von der Austragsvorrichtung abgenommen werden. Zum eigentlichen Austragen muss jedoch wiederum eine Austragsspitze auf die Vorrichtung angebracht werden, sodass hier ein Benutzer die Austragvorrichtung nur mit erhöhten Zeit-und Montageaufwand verwenden kann.

WO 2009/036962 A2 und DE 10 2007 044983 A1 beschreiben eine Austragvorrichtung mit einem Applikator, der lösbar an einem Behälter befestigt ist. Um ein unbeabsichtigtes Ablösen des Applikators zu verhindern, ist ein Verriegelungsring vorgesehen, welcher zusammen mit einem Federarm eine Kupplungseinrichtung bildet, die den Applikator am Behälter festhalten kann. Der Verriegelungsring ist von einer den Applikator freigebenden Position in eine den Applikator an dem Behälter verriegelnden Position drehbar. Um die im Behälter aufgenommenen Komponenten vor Umwelteinflüssen zu schützen, ist ein Verschlussstopfen vorgesehen, welcher durch den Druck der auszubringenden Komponenten selbsttätig öffnet.

Die vorliegende Erfindung stellt sich die Aufgabe, einen Behälter der eingangs genannten Art bereitzustellen, der eine besonders sichere und dichte Aufbewahrung der Komponenten, unbeeinflusst von äußeren Bedingungen, wie Temperatur- oder Druckschwankungen, mechanischen Transportbelastungen (Rütteln, Fallen, etc.) bei gleichzeitig hoher Bedienerfreundlichkeit ermöglicht. Der Behälter soll zudem eine fehlervermeidende Anwendung erlauben, indem eine versehentliche, unbewusste Aktivierung vermieden wird und ein aktives Bereitstellen des Behälters ermöglicht wird, wenn der Inhalt des Behälters noch dicht verschlossen ist.

Diese Aufgabe wird mit einem Behälter mit den Merkmalen des Anspruchs 1 gelöst. Ein Kerngedanke der Erfindung liegt in einem zweistufigen Öffnungsvorgang des Behälters, wobei in einem ersten Schritt, insbesondere durch eine relative Drehung des Applikators zu dem Behälter, ein Bereitstellungszustand erreicht wird und in einem zweiten Schritt, insbesondere durch eine axiale Bewegung des Stopfens relativ zu dem Behälter und/oder dem Applikator, eine Strömungsverbindung zwischen der wenigstens einen Kammer und dem Applikator hergestellt wird. In dem Bereitstellungszustand ist die wenigstens eine Kammer dabei zwar noch von dem Stopfen verschlossen, aber der Stopfen kann, z.B. durch den Innendruck der Komponente in der Kammer, jederzeit verschoben werden, um die Strömungsverbindung herzustellen. Dagegen wird vor dem ersten Schritt, d.h. in einem Transport- oder Lagerzustand des Behälters, eine axiale Bewegung des Stopfens - und damit die Herstellung einer Strömungsverbindung zwischen der wenigstens einen Kammer und dem Applikator - verhindert. Somit wird ein unbeabsichtigtes Öffnen des Behälters, z.B. durch Temperatur- oder Druckschwankungen oder mechanischen Transportbelastungen, in dessen Transport- oder Lagerzustand aktiv verhindert.

Mit anderen Worten wird der Behälter in einem Transport- oder Lagerzustand bereitgestellt, in dem der Behälter sicher verschlossen ist, und kann durch eine Drehung des Applikators zu dem Behälter in einen Bereitstellungszustand überführt werden, in dem der Behälter zwar noch geschlossen ist, aber ohne weitere Schritte allein durch den beim Ausbringen der Komponenten auf diese ausgeübten Druck, der eine axiale Bewegung des Stopfens bewirkt, vollständig geöffnet werden kann, so dass die Komponenten aus dem Applikator austreten können. Die Erfindung zeichnet sich dabei im Wesentlichen dadurch aus, dass der die Kammer(n) verschließende Stopfen in einer Lagerposition eines Applikators verschiebesicher gehalten wird, während in einer Bereitstellungsposition des Applikators der Stopfen aus seiner Lagerposition, in der eine Strömungsverbindung zwischen der Kammer und dem Applikator durch den Stopfen unterbrochen ist, in seine Ausbringposition, in der die Kammer mit dem Applikator in Strömungsverbindung steht, axial verschoben werden kann, und dass der Applikator von der Lagerposition durch Drehung in die Bereitstellungsposition überführt werden kann. Mit anderen Worten basiert die Erfindung daher auf dem Gedanken, dass der Stopfen so lange in seiner Lagerposition festgehalten wird, bis der Applikator aus der Lagerposition in die Bereitstellungsposition bewegt wird, bspw. durch eine Drehbewegung des Applikators. Erst in dieser Bereitstellungsposition des Applikators kann dann der Stopfen zur Freigabe von Strömungskanälen für die im Behälter gelagerten Komponenten ebenfalls in seine Ausbringposition bewegt werden. Damit wird ein unbeabsichtigtes Öffnen des Behälters z.B. während Transport und/oder Lagerung verhindert und auch nachdem der Applikator in die Bereitstellungsposition überführt ist, ist der Behälter noch durch den Stopfen verschlossen, kann jedoch durch eine Bewegung des Stopfens jederzeit geöffnet werden. Der Applikator wirkt somit wie eine Transportsicherung für den Stopfen. Trotz dieser gegenüber bekannten Behältern verbesserten Sicherheit während Transport und/oder Lagerung ist der Behälter schnell und ohne aufwendige Montageschritte einsatzbereit, indem lediglich der im Auslieferungszustand des Behälters vormontierte Applikator in die Bereitstellungsposition überführt wird. Eine Drehbewegung, die den Applikator aus seiner Lagerposition in die Bereitstellungsposition überführt, ist für einen Benutzer von außen gesehen eine Relativbewegung in Umfangsrichtung zwischen Applikator und Behälter, relevant für das Öffnen des Behälters nach dem Freigeben des Stopfens (d.h. seine Ausbringposition) ist jedoch eine axiale Relativbewegung zwischen dem Applikator und dem Stopfen.

In der Lagerposition des Stopfens ist eine Strömungsverbindung zwischen der Kammer und dem Applikator durch den Stopfen unterbrochen, während in der Ausbringposition des Stopfens die Kammer mit dem Applikator in Strömungsverbindung steht. Um ein Verschieben des Stopfens in der Lagerposition zu verhindern, weist der Stopfen ein erstes Sperrelement und der Applikator ein zweites Sperrelement auf, welche in der Lagerposition so zueinander ausgerichtet sind, dass diese ein Verschieben des Stopfen verhindern.

Der Stopfen liegt dabei in der Lagerposition nicht innerhalb der Kammer(n), sondern ist in einem z.B. ringförmigen Raum des Behälters aufgenommen. Der Stopfen weist jedoch Dichtmittel auf, die eine Strömungsverbindung zwischen der Kammer und dem Applikator sperren, wenn der Stopfen in der Lagerposition ist. Diese Dichtmittel können umfangsseitige und/oder stirnseitige proximale Dichtungen des z.B. im Wesentlichen kreisringförmigen Stopfens aufweisen und/oder wenigstens einen proximalen Vorsprung, der einen Ausbringkanal der jeweiligen Kammer verschließt.

Mit der Erfindung ist es demnach möglich den Behälter, insbesondere durch Verdrehen des Applikators, in die Bereitstellungsposition zum Austragen der wenigstens einen Komponente vorzubereiten, ohne dass der Behälter bereits geöffnet ist und die darin gelagerte Komponente bereits Umwelteinflüssen, bspw. Luft, ausgesetzt wird, da der Stopfen noch nicht in seine Ausbringposition verschoben ist. Mit anderen Worten sind die Bereitstellungsposition des Applikators und die Ausbringposition des Stopfens von einander entkoppelt, also insofern unabhängig voneinander, dass der Stopfen in der Bereitstellungsposition des Applikators nicht in seiner Ausbringposition vorliegen muss. Allerdings kann der Stopfen nur dann in seine Ausbringposition verschoben werden, wenn der Applikator bereits in seine Bereitstellungsposition verdreht wurde. Damit wird ein vorbereitendes Öffnen des Behälters, bspw. durch einen Assistenten eines behandelnden Zahnarztes, ermöglicht, ohne dass auch bei anschließender längerer Nicht-Benutzung des Behälters, z.B. während einer Behandlungswartezeit, eine Kontamination der wenigstens einen Komponente stattfindet.

Ein erfindungsgemäßer Behälter kann sowohl mit einer einzelnen Kammer als so genanntes Ein-Komponentensystem als auch mit mehreren Kammern als so genanntes Mehr-Komponentensystem ausgebildet sein. Insbesondere Zwei-Komponentensysteme, welche zwei typischerweise zu mischende Substanzen umfassen, werden häufig verwendet. Dabei können die Kammern zur Aufnahme der beiden Komponenten als parallel angeordnete Zylinder nebeneinander, bspw. jeweils halbkreisförmig, oder auch ineinander, bspw. konzentrisch, ausgebildet sein.

Der verwendete Applikator kann ein Mischer, eine ggf. gebogene Kanüle, insbesondere eine Metallkanüle, eine Bürste oder ein Schwamm sein. Dabei ist ein Mischer für Mehr-Komponentensysteme mit gebogener Kanüle oder Metallkanüle bevorzugt, während einfachere Applikatoren, wie eine Kanüle, für Ein-Komponentensystem bevorzugt sind.

Metallkanülen werden insbesondere beim Austragen z.B. hochviskoser Retraktionspasten oder dünnviskoser fließfähiger Dentalfüllungscomposite oder schnellaushärtender Materialien eingesetzt, insbesondere für den Anwender ergeben sich Handhabungsvorteile, da die Metallnadel individuell für die jeweilige Mundsituation biegsam ist, und da diese oftmals mechanisch stabiler sind und rückstellfrei biegsam sind. Aufgrund der im Vergleich zu Kunststoffmaterialien erhöhten Stabilität können bei gleichem Außendurchmesser die Metallnadeln mit höherem Innendurchmesser ausgebildet werden, was insbesondere bei schnell aushärtenden Materialien vorteilhaft ist, da ein Verstopfen der Kanüle verhindert oder zumindest verzögert wird bzw. die Ausdrückkraft reduziert wird. Insbesondere bei korrosiven und/oder ätzenden Materialien, z.B. Retraktionspasten, Ätzgele, Zemente und Adhäsive, kommt es immer wieder zur Korrosion der werksmäßig aufgesetzten Metallnadeln. Dieses Problem wird mit der vorliegenden Erfindung gelöst, da die Dichtwirkung des Stopfens durch das Zusammenwirken der ersten und zweiten Sperrelemente derart hoch ist, dass auch bei vom Hersteller werksmäßig vormontierten Metallnadeln auch nach längerer Lagerung keine Korrosion auftritt.

Der erfindungsgemäße Behälter ist vorzugsweise in eine konventionelle Ausbringpistole (bspw. eine Pistole der Firma Centrix oder der Firma Ronvig-Dental) einlegbar. Zum Festlegen des Behälters in der Ausbringpistole weist dieser einen entsprechend ausgestalteten Halteabschnitt auf. Ist der Behälter, bspw. eine Kapsel, in der Ausbringpistole eingelegt, kann durch Betätigen des Stößels der Ausbringpistole die wenigstens eine Komponente ausgebracht werden, wenn der Applikator in der Bereitstellungsposition ist und der Stopfen in der Ausbringposition ist.

Es wird bevorzugt, wenn der Stopfen in der Bereitstellungsposition des Applikators durch den Innendruck der wenigstens einen Komponente in die Ausbringposition des Stopfens verschiebbar ist. Demnach ist ein intuitives, automatisches Öffnen des Behälters, bspw. durch Betätigung der Ausbringpistole, möglich, wobei die wenigstens eine Komponente in Richtung des Applikators gedrückt wird und dadurch den Stopfen in die Ausbringposition verschiebt. Dadurch ist es möglich, dass nach der Drehung des Applikators relativ zu dem Behälter in dessen Bereitstellungsposition der Benutzer lediglich die Ausbringpistole betätigen muss, um die Komponenten auszutragen, während gleichzeitig eine Kontamination der Komponenten vor dem eigentlichen Ausbringen der wenigstens einen Komponente verhindert wird.

Das zweite (proximale) dem Applikator abgewandte Ende der wenigstens einen Kammer ist vorzugsweise mit einem Kolben verschlossen, an welchem ein Stößel einer Ausbringpistole ansetzen kann, um durch Verschieben des Kolbens die wenigstens eine Komponente auszubringen. Alternativ ist auch eine Dichtungsfolie am proximalen Ende der Kammer zum Verschließen der Kammer möglich, welche über einen Kolben, insbesondere mit Kolbenstange, beim Ausbringen der wenigstens einen Komponente durchstoßen werden kann. In einer weiteren Alternative kann die Kammer an ihrem proximalen Ende vollständig verschlossen sein, wobei die wenigstens eine Komponente z.B. durch Gasdruck ausgebracht wird.

In einer bevorzugten Variante wird der Applikator in der Lagerposition und in der Bereitstellungsposition jeweils axial festgelegt, ist also nicht axial relativ zu der Kammer bewegbar. Dies schließt nicht aus, dass eine axiale Bewegungskomponente auftritt, während der Applikator aus der Lagerposition in die Bereitstellungsposition überführt wird. Da der Applikator in der Lagerposition axial festgelegt ist, wird ein unbeabsichtigtes Freigeben des Stopfens in der Lagerposition durch ein Verschieben des Applikators verhindert. Auch Druck- und/oder Temperaturschwankungen sowie bei der Lagerung und/oder dem Transport auftretende mechanische Belastungen, bspw. Erschütterungen oder Rüttelbewegungen, führen nicht zu einem unbeabsichtigtem Freistellen des Stopfens und damit nicht zu einem Ausbringen der Komponenten. Ist der Applikator auch in der Bereitstellungsposition nicht axial verschiebbar, können auch hohen Ausbringdrücke, bspw. durch die Ausringpistole, aufgebracht werden.

Es ist weiter bevorzugt, wenn der Applikator aus einer Montageposition, in der der Applikator axial auf den Behälter aufsteckbar ist, relativ zu der Kammer in die Lagerposition und in die Bereitstellungsposition drehbar ist. Vorzugsweise ist die Drehrichtung vorgegeben, sodass der Applikator aus der Montageposition in die Lagerposition und aus der Lagerposition in die Bereitstellungsposition drehbar ist. Der zu drehende Winkel zwischen den einzelnen Positionen kann bspw. jeweils etwa 30 bis 120° und damit insgesamt etwa 60 bis 240°, bevorzugt 30 bis 90° und damit insgesamt 60 bis 180° und insbesondere bevorzugt 45 bis 60° und damit insgesamt 90 bis 120°, betragen. Bevorzugt ist eine Drehung jeweils um 45°. Die Montageposition ist dabei eine z.B. während der Montage vor der Auslieferung an den Benutzer auftretende Position, in der der Applikator mit dem Behälter verbunden wird. Die Verbindung zwischen dem Applikator und dem Behälter kann dabei so gestaltet werden, dass der Applikator aus der Lagerposition nicht oder nicht zerstörungsfrei wieder zurück in die Montageposition bewegt werden kann. Damit ist der Applikator in seinem Auslieferungszustand an einen Benutzer, d.h. in seiner Lagerposition, verliersicher auf dem Behälter befestigt.

Besonders zweckmäßig ist es bei dem Behälter eine Aufnahme für den Applikator vorzusehen, welche wenigstens einen radial nach innen gerichteten Bajonettvorsprung aufweist. Dabei weist der Applikator einen in die Aufnahme einsteckbaren Anschlussabschnitt mit wenigstens einem radial nach außen gerichteten Bajonettvorsprung auf. Um ein Verkanten des Applikators zu vermeiden sind jeweils vorzugsweise zwei, drei oder mehr, Bajonettvorsprünge vorgesehen. Die Bajonettvorsprünge sind relativ zueinander derart angeordnet, dass diese in der Montageposition ein Aufstecken des Applikators auf den Behälter ermöglichen. Wird der Applikator in die Lagerposition verdreht, überdecken, also hintergreifen, die entsprechenden Bajonettvorsprünge des Anschlussabschnitts des Applikators und der Aufnahme des Behälters einander zumindest teilweise. Überdecken die entsprechenden Bajonettvorsprünge einander auch in der Bereitstellungsposition kann ein axiales Verschieben des Applikators verhindert werden. Vorzugsweise ist die sich überdeckende bzw. hintergreifende Fläche der Bajonettvorsprünge in der Bereitstellungsposition größer als in der Lagerposition. Es wird bevorzugt, wenn die entsprechenden Bajonettvorsprünge in der Bereitstellungsposition einander vollständig überdecken, sofern die Bajonettvorsprünge von Applikator und Behälter gleich lang ausgeführt werden. Werden die Bajonettvorsprünge in der Aufnahme und/oder dem Anschlussabschnitt unterschiedlich lang ausgeführt, ist ein vollständiges Überdecken bzw. Hintergreifen eines, bspw. des kürzeren, Bajonettvorsprungs in der Bereitstellungsposition bevorzugt. Alternativ zu dem in die Aufnahme des Behälters einsteckbaren Applikator kann der Applikator eine Aufnahme aufweisen, in die der Behälter einsteckbar ist.

Dabei ist vorteilhaft, dass beim Ausbringen der wenigstens einen Komponente ein vollständiges, Überdecken bzw. Hintergreifen der entsprechenden Bajonettvorsprünge eine hohe Kraftübertragung ermöglicht und der Applikator daher auch bei hohem Ausbringdruck sicher an dem Behälter festgelegt ist.

In einer weiteren Ausgestaltung der Erfindung sind die Bajonettvorsprünge der Aufnahme des Behälters und/oder des Anschlussabschnitts des Applikators zumindest teilweise mit Schrägen ausgebildet, welche beim Verdrehen des Applikators in die Lagerposition und/oder die Bereitstellungsposition ein Heranziehen des Applikators auf den Behälter erreichen. Dadurch wird eine besonders gute Abdichtung zwischen Applikator und Behälter erreicht.

Weiter wird es bevorzugt, wenn der Behälter auf seinem der Aufnahme für den Applikator gegenüberliegenden (proximalen) Ende einen flanschartigen Halteabschnitt mit vergrößertem Außendurchmesser aufweist. Dieser Halteabschnitt kann der Befestigung innerhalb einer Ausbringpistole dienen oder zur Verwendung des Behälters als ein Spritze.

Nach einer bevorzugten Ausführungsform der Erfindung sind an Behälter und/oder dem Applikator Rastmittel vorgesehen sind, die eine relative Drehbewegung des Applikators zu dem Behälter in einer ersten Drehrichtung erlauben und eine Drehbewegung in einer anderen, insbesondere entgegengesetzten, Drehrichtung verhindern. Mit anderen Worten wird die Drehbewegung in der ersten Drehrichtung wenig oder überhaupt nicht von den Rastmitteln beeinflusst, während die Drehbewegung in der anderen Drehrichtung soweit erschwert wird, dass diese nicht mehr händisch, also ohne Hilfsmittel, vom Benutzer möglich ist. Besonders bevorzugt ist eine Drehbewegung in der anderen Drehrichtung nicht ohne eine Beschädigung oder Zerstörung des Behälters und/oder Applikators möglich.

Besonders bevorzugt ist es, wenn die Rastmittel eine relative Drehbewegung zwischen Applikator und Behälter erschweren. Dabei ist von Vorteil, dass ein unbeabsichtigtes Verdrehen des Applikators in dessen Bereitstellungsposition verhindert oder zumindest erschwert wird. Insbesondere kann durch die Rastmittel auch eine bestimmte Drehrichtung, bspw. von der Befestigungs- in die Lagerungsposition und/oder von der Lagerungs- in die Bereitstellungsposition, durch erhöhten Drehwiderstand vorgegeben werden.

Diese Funktionen können bspw. dadurch erreicht werden, dass die Rastmittel am Applikator einen Vorsprung bilden, welche in am Behälter vorgesehene Rastmittel, z.B. Rastausnehmungen, eingreifen, wenn der Applikator mit dem Behälter verbunden ist, also in der Montage-, Lagerungs- und/oder Bereitstellungsposition. Um ein relatives Drehen zwischen Applikator und Behälter zu erschweren, kann die Form der Rastmittel angepasst werden. Dabei ist es bevorzugt, die Rastmittel im Wesentlichen als halbkreisförmige Aussparungen am Behälter vorzusehen. Wird der Applikator verdreht, so muss das an diesem festgelegte Rastmittel aus der Aussparung herausgedreht werden. Dafür ist eine Drehung über eine Ecke der Aussparung hinweg und eine entsprechend aufzuwendende Kraft notwendig. Soll die Drehung des Applikators nur in einer Drehrichtung erschwert werden, können die im Wesentlichen rechtwinkligen Ecken der halbkreisförmigen Aussparungen wenig oder nicht abgerundet werden. Je weniger die Ecken der halbkreisförmigen Aussparungen abgerundet werden, desto schwerer ist ein Verdrehen des Rastmittels des Applikators über diese Ecke hinweg. Entsprechend kann eine Drehrichtung durch eine deutliche Abrundung der Ecken der Rastmittel bevorzugt werden, sodass für eine Drehung in diese Drehrichtung weniger Kraft aufgewendet werden muss und die Drehung in dieser Richtung erleichtert wird.

In einer bevorzugten Ausführung der Erfindung ist das erste am Stopfen vorgesehene Sperrelement ein Steg oder ein Vorsprung und das zweite am Applikator vorgesehene Sperrelement ein Steg oder ein Vorsprung, welcher zumindest in der Lagerposition den Steg oder Vorsprung des Stopfens überdeckt. Dabei kann der Vorsprung des ersten Sperrelements am Stopfen distale (dem Applikator zugewandte) Kontaktflächen aufweisen und das zweite Sperrelement (am Applikator) proximale Kontaktflächen ausweisen, wobei die distalen und proximalen Kontaktflächen einander zugewandt sind und in der Lagerposition aufeinander liegen.

Besonders bevorzugt sind kreuzförmige Stege als erstes Sperrelement vorgesehen. Die zweiten Sperrelemente sind vorzugsweise als in etwa viertelkreisförmige Blöcke innerhalb des Anschlussabschnitts des Applikators ausgebildet. Die mindestens zwei, vorzugsweise vier, Blöcke sind auf einer Kreisbahn äquidistant angeordnet und zwischen den Blöcken sind Aussparungen vorgesehen, welche in der Bereitstellungsposition des Applikators die ersten Sperrelemente, bspw. die Stege, aufnehmen können. Das können auch den ersten Sperrelementen entsprechend nachgebildete Schlitze sein, sodass nur bei einer exakten Positionierung des Applikators in die Bereitstellungsposition ein Verschieben des Stopfens ermöglicht wird. Vorteilhaft an den oben beschriebenen Ausgestaltungen ist die besonders stabile Festlegung des Stopfens in der Lager- und Ausbringposition.

Alternativ zu der oben beschriebenen bevorzugten Ausgestaltung der Erfindung können die Sperrelemente auch als seitlich vorgesehener Vorsprung bzw. Nut ausgebildet sein. So kann das erste Sperrelement als seitlich an dem Stopfen ausgebildeter Bajonettvorsprung vorgesehen werden. In dieser Ausgestaltung der Erfindung ist das zweite Sperrelement als eine im Inneren des Anschlussabschnitts des Applikators bereichsweise umlaufende und bereichsweise axial verlaufende Bajonettnut zur Aufnahme des Bajonettvorsprungs des Stopfens in der Bereitstellungsposition des Applikators vorgesehen. Damit kann nur bei einer exakten Positionierung des Applikators in dessen Bereitstellungsposition der Stopfen verschoben werden.

In einer weiteren Ausführungsform der Erfindung, sind die ersten und zweiten Sperrelemente derart ausgestaltet, dass der Stopfen auch in der Montageposition des Applikators relativ zum Applikator nicht verschiebbar ist. Dabei kann der Applikator beim Befestigen an dem Behälter den Stopfen auch in die Kammern eindrücken, sodass eine eventuell nicht richtige oder unvollständige Montage des Stopfens in den Behälter automatisch durch Befestigen des Applikators korrigiert werden würde.

Es ist weiterhin bevorzugt, wenn der Stopfen wenigstens einen (inneren oder äußeren) Bypasskanal aufweist, durch den in seiner Ausbringposition eine Strömungsverbindung von Kammer zu Applikator hergestellt wird. Dazu kann bspw. eine ringförmige, axial durchgängige Aussparung im Stopfen vorgesehen werden, welche zur festen Verbindung der von der Aussparung getrennten Abschnitte mit Stegen unterbrochen ist. Werden bspw. zwei solche Stege vorgesehen sind vier Öffnungen innerhalb der Aussparung ausgebildet durch welche die wenigstens eine Komponente in Richtung Applikator strömen und ausgebracht werden kann.

Dazu ist es zweckmäßig, wenn die wenigstens eine Kammer wenigstens eine Auslassöffnung aufweist, und dass der Stopfen wenigstens einen Verschlussabschnitt aufweist, der in der Lagerposition des Stopfens die Auslassöffnung verschließend in diese eingesteckt ist. Sind mehrere Kammern vorgesehen, weisen diese jeweils mindestens eine Auslassöffnung auf, in welche ein Verschlussabschnitt des Stopfens diese verschließend eingesteckt ist. Im Vergleich zu einem die Auslassöffnungen lediglich abdeckenden, im Wesentlichen ebenen Stopfen wird durch eine solche Ausbildung des Stopfens eine erhöhte Dichtigkeit erreicht. Insbesondere wird ein unbeabsichtigtes Vermischen der Komponenten im verschlossenen Zustand des Behälters praktisch unmöglich, da die Komponenten dazu nicht nur den Stopfen verschieben, sondern auch um mehrere durch die Verschlussabschnitte gebildete Ecken (so genannte Verschlusskanten) strömen müssten.

Der Stopfen ist gemäß einer besonders bevorzugten Ausführung der Erfindung in der Lagerposition und ggf. auch in der Ausbringposition drehfest, verkippfest und verkantfest in dem Behälter geführt. Hierdurch wird in der Lagerposition verhindert, dass der Stopfen ohne ein Überführen des Applikators in seine Bereitstellungsposition so verdreht wird, dass der Stopfen eine Stellung relativ zu dem Applikator einnimmt, die es ermöglichen würde, den Stopfen zu verschieben. Dies kann durch entsprechende Kodierungsmittel am Stopfen erreicht werden, die in dafür vorgesehene Aussparungen innerhalb des Gehäuses des Behälters eingreifen, welche sowohl eine drehfeste Führung in der Lagerposition und, bei entsprechender Länge der Kodierungsmittel, bis in der Ausbringposition des Stopfens erlauben. In der Ausbringposition ist es auch möglich, ein Verdrehen des Stopfens durch entsprechende Ausgestaltung der ersten und zweiten Sperrelemente zu verhindern, bspw. indem die ersten Sperrelemente in für diese vorgesehenen Aussparungen zwischen den zweiten Sperrelementen geführt werden.

Beispielsweise kann der Stopfen eine im Wesentlichen kreisscheibenförmige Grundform und auf seiner der wenigstens einen Kammer zugewandten (proximalen) Seite und/oder an seinem Außenumfang Dichtmittel aufweisen. Auf seiner der wenigstens einen Kammer abgewandten (distalen) Seite kann ein durch zwei zueinander rechtwinklig verlaufende Stege gebildetes Sperrelement vorgesehen sein. Weiter weist der Stopfen vorzugsweise wenigstens einen Durchbruch als Bypasskanal auf.

Die wenigstens eine Kammer ist auf ihrer dem Stopfen abgewandten (proximalen) Seite mit einem Kolben verschlossen. Der Kolben ist in der jeweiligen Kammer axial verschiebbar. Nach einer besonders bevorzugten Ausführungsform ist der Kolben über wenigstens eine Sollbruchstelle einstückig mit dem Behälter verbunden. Dies erleichtert die Montage des Behälters. Der Kolben kann z.B. vor dem befüllen des Behälters oder währenddessen von dem Behälter abgetrennt werden, um in der Kammer frei verschiebbar zu sein.

Zusätzlich kann der Behälter mit einem separaten Stößel ausgebildet sein, der zum Einstecken in die wenigstens eine Kammer und zum Verschieben des Kolbens geeignet ist. Für Behälter mit zwei konzentrisch angeordneten Kammern ist ein distaler Abschnitt des Stößels mit einem äußeren Zylinder für den äußeren Kolben und einem zentralen Stift für den inneren Kolben ausgestaltet. Es kann vorteilhaft sein, wenn der Stift gegenüber dem Zylinder vorspringt oder zurückgesetzt ist, um das z.B. durch unterschiedliche Viskositäten verursachte Voreilen einer der Komponenten beim Ausbringen zu verhindern oder jedenfalls zu reduzieren.

Der Behälter kann auch spritzenartig mit einer Auflagefläche für Finger und einem entsprechenden Gegenlager am Stößel ausgebildet sein. Dies ermöglicht es, Komponenten ohne eine Ausbringpistole manuell aus dem Behälter auszutragen.

Neben dem Vorteil, dass ein unbeabsichtigtes Öffnen des Stopfens durch eine Drehung in der Lagerposition relativ zu dem Applikator verhindert wird, wird der Stopfen zudem beim Verschieben in die Ausbringposition derart geführt, dass ein Verkippen oder Verkanten desselben im Behälter verhindert wird, was sonst zu Problemen beim Austragen der wenigstens einen Komponente führen kann, bspw. indem es zu einer Blockade der Strömungsverbindung zwischen Behälter und Applikator kommt oder indem lediglich ein Austragkanal freigegeben wird, sodass das Mischungsverhältnis zweier Komponenten gestört wird.

Die der Erfindung zugrundeliegende Aufgabe wird auch durch ein Verfahren zum Ausbringen wenigstens einer Komponente aus einem Behälter, bspw. einem wie oben beschriebenen Behälter, gelöst. Der Behälter weist hierzu wenigstens eine Kammer auf, die auf einer ersten Seite von einem Stopfen verschlossen ist, und mit einem auf der ersten Seite drehbar befestigten Applikator, der in einer Lagerposition eine axiale Bewegung des Stopfens verhindert. Dabei weist das Verfahren folgende Schritte auf: Drehen des Applikators relativ zu dem Behälter in eine Bereitstellungsposition, in der der Applikator eine axiale Bewegung des Stopfens nicht verhindert, und anschließendes Verschieben des Stopfens, vorzugsweise durch den Innendruck der wenigstens einen Komponente, aus einer Lagerposition, in der eine Strömungsverbindung zwischen der Kammer und dem Applikator durch den Stopfen unterbrochen ist, in eine Ausbringposition, in der die Kammer mit dem Applikator in Strömungsverbindung steht.

Nach einer bevorzugten Ausführungsform des Verfahrens wird Drehen des Applikators durch an dem Behälter und/oder dem Applikator vorgesehene Rastmittel in einer ersten Drehrichtung erlaubt und in einer anderen Drehrichtung verhindert. Mit anderen Worten wird Drehen in der ersten Drehrichtung wenig oder überhaupt nicht von den Rastmitteln beeinflusst, während Drehen in der anderen, insbesondere entgegengesetzten, Drehrichtung soweit erschwert wird, dass diese nicht mehr händisch, also ohne Hilfsmittel, vom Benutzer durchgeführt werden kann. Besonders bevorzugt ist Drehen in der anderen Drehrichtung nicht ohne eine Beschädigung oder Zerstörung des Behälters und/oder Applikators möglich.

Vor dem Austragen der wenigstens einen Komponente wird der Behälter montiert und mit der wenigstens einen Komponente befüllt. Dazu wird zunächst der Kolben in die wenigstens eine Kammer eingesetzt und bis zum distalen Ende der Kammer geführt. Über dieses Ende kann dann die Kammer bei Verschiebung des Kolbens in proximaler Richtung befüllt werden. Anschließend wird der Stopfen zum Abdichten der wenigstens einen Kammer aufgesetzt. Schließlich wird der Applikator in der Montageposition am Behälter befestigt und insbesondere bereits vom Hersteller werksmäßig in die Lagerposition gedreht. Der Behälter ist dadurch dicht abgeschlossen und die Komponenten können sicher aufbewahrt werden. Vorzugsweise wird der Behälter in der Lagerposition gelagert und ausgeliefert. Der Benutzer des Behälters, welcher die Komponenten austragen muss, kann den Applikator in dessen Bereitstellungsposition durch relative Drehung zum Behälter überführen. In dieser Position ist der Behälter noch nicht geöffnet. Vielmehr wird erst, insbesondere durch den Innendruck der Komponenten, nach Verschieben des Stopfens eine Strömungsverbindung zwischen den Kammern und dem Applikator hergestellt, sodass Komponenten ausgetragen werden können.

Damit weist ein erfindungsgemäßer Behälter in seiner bevorzugten Ausgestaltung eine Montage-, eine Lager- und eine Bereitstellungsposition für den Applikator sowie eine Lager- und eine Ausbringposition für den Stopfen auf, wobei die Ausbringposition des Stopfens und die Bereitstellungsposition des Applikators voneinander derart entkoppelt sind, dass der Stopfen in der Bereitstellungsposition des Applikators zunächst in seiner Lagerposition die wenigstens eine Kammer dichtend abschließt.

Die Erfindung wird nachfolgend anhand von Ausführungsbeispielen und unter Bezugnahme auf die Zeichnungen näher erläutert. Dabei bilden alle beschriebenen und/oder bildlich dargestellten Merkmale für sich oder in beliebiger Kombination den Gegenstand der Erfindung unabhängig von ihrer Zusammenfassung in den Ansprüchen oder deren Rückbeziehungen.

Es zeigen schematisch:
- Fig. 1: einen Längsschnitt eines erfindungsgemäßen Behälters nach einer ersten Ausführungsform,
- Fig. 2: eine Explosionsansicht der Bauteile des Behälters nach Fig. 1,
- Fig. 3: eine Explosionsansicht der Bauteile eines erfindungsgemäßen Behälters nach einer zweiten Ausführungsform,
- Fig. 4a: einen Längsschnitt des Behälters nach Fig. 3 in der Montageposition des Applikators,
- Fig. 4b: eine Draufsicht des Behälters nach Fig. 4a,
- Fig. 4c: eine Draufsicht des Behälters nach Fig. 4a ohne Applikator,
- Fig. 4d: eine teilweise aufgebrochene Ansicht eines Details des Behälters nach Fig. 4a,
- Fig. 5a: eine Schnittansicht des Behälters nach Fig. 3 in der Lagerposition des Applikators,
- Fig. 5b: eine Draufsicht des Behälters nach Fig. 5a,
- Fig. 5c: eine teilweise aufgebrochene Ansicht eines Details des Behälters nach Fig. 5a,
- Fig. 6a: eine Schnittansicht des Behälters nach Fig. 3 in der Bereitstellungsposition des Applikators und der Ausbringposition des Stopfens,
- Fig. 6b: eine Draufsicht des Behälters nach Fig. 6a,
- Fig. 6c: eine teilweise aufgebrochene Ansicht eines Details des Behälters nach Fig. 6a,
- Fig. 7: den Behälter der zweiten Ausführungsform sowie zwei weitere erfindungsgemäße Applikatoren,
- Fig. 8a - c: den Stopfen aus Fig. 2 in Draufsicht, in Längsschnitt und in Perspektivansicht,
- Fig. 9a - c: den Stopfen aus Fig. 3 in Draufsicht, in Längsschnitt und in Perspektivansicht,
- Fig. 10a - c: den Kolben aus Fig. 2 in Perspektivansicht, in Draufsicht und in Längsschnitt,
- Fig. 11a - c den: Kolben aus Fig. 3 in Perspektivansicht, in Draufsicht und in Längsschnitt,
- Fig. 12: in Seitenansicht eine Pistole zur Verwendung mit einem Behälter nach Fig. 1 oder 3,
- Fig. 13a - b: einen Stößel der Pistole nach Fig. 12 in Draufsicht und in Längsschnitt,
- Fig. 14a - b: einen alternativen Stößel der Pistole nach Fig. 12 in Draufsicht und in Längsschnitt,
- Fig. 15a - c: eine weitere Ausführungsform eines erfindungsgemäßen Behälters in Perspektivansicht, in Explosionsansicht und in Längsschnitt, und
- Fig. 16a - b: in teilweise aufgebrochener Darstellung den Strömungsverlauf der Komponenten im geöffneten Zustand des Behälters nach Fig. 1 bzw. nach Fig. 3.

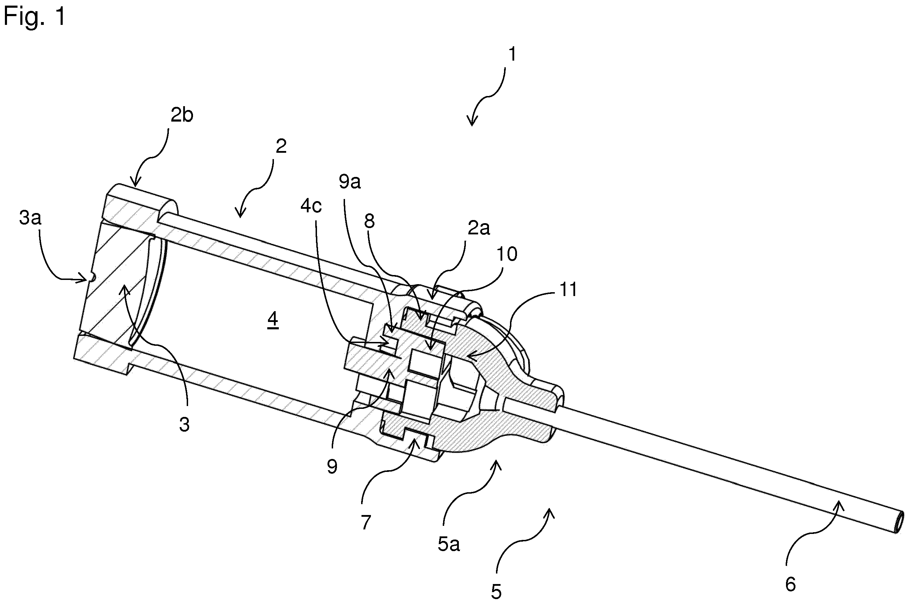

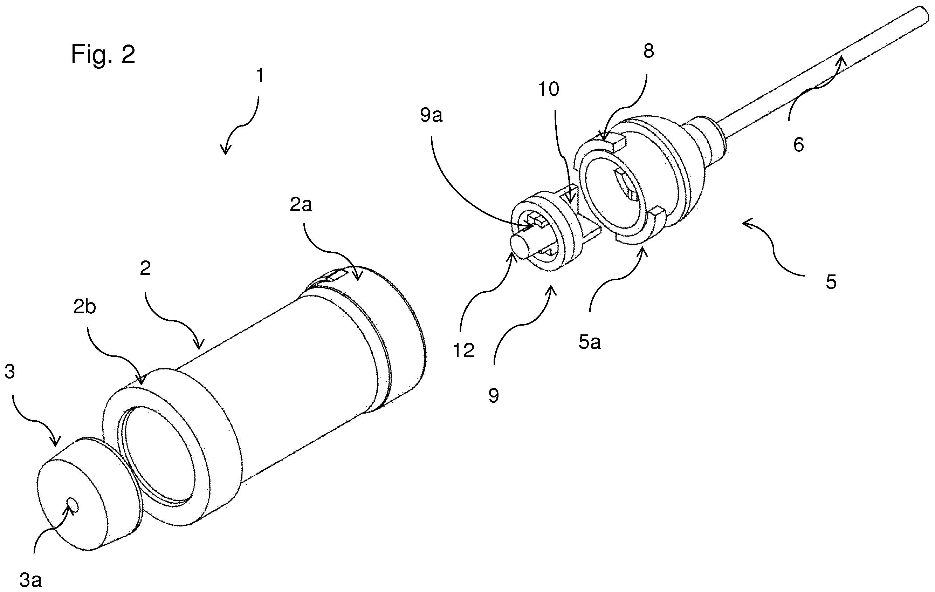

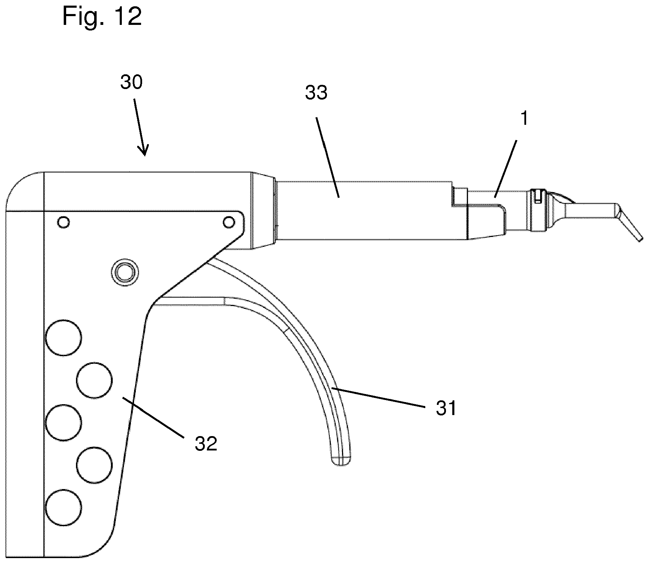

In den Fig. 1 und 2 ist ein erfindungsgemäßer Behälter 1 dargestellt. Der Behälter 1 ist mit einem innen und außen im Wesentlichen zylindrischen Gehäuse 2 ausgebildet, welches am proximalen Ende durch einen Kolben 3 abgeschlossen ist. Innerhalb des Gehäuses 2 ist in dieser Ausführungsform nur eine Kammer 4 für die Aufnahme einer Komponente ausgebildet. Der Kolben 3 kann, bspw. mittels einer in Fig. 12 dargestellten Pistole 30, in Richtung der ersten (distalen) Seite verschoben werden, d.h. nach rechts in Fig. 1, wodurch das Volumen der Kammer 4 definiert verringert wird. Zur Verbesserung der mechanischen Verbindung eines Stößels einer Ausbringpistole mit dem Kolben 3 weist dieser vorzugsweise eine Einkerbung 3a auf, welche die Spitze des Stößels der Ausbringpistole teilweise aufnehmen kann und so ein Verrutschen des Stößels verhindert oder zumindest erschwert.

An dem Behälter 1 ist ein Applikator 5, in der Ausführungsform nach Fig. 1 mit einer Kanüle 6 versehen, befestigt. Hierzu weist der Behälter 1 auf der dem Applikator 5 zugewandten ersten (distalen) Seite eine hülsenartige Aufnahme 2a zur Befestigung eines Applikators 5 mit radial nach innen gerichteten Bajonettvorsprüngen 7 auf. Der Applikator 5 weist einen sockelartigen Anschlussabschnitt 5a mit radial nach außen gerichteten Bajonettvorsprüngen 8 auf. Die Bajonettvorsprünge 7 und 8 erlauben den Applikator 5 in einer Montageposition auf dem Behälter 1 zu montieren und den Applikator 5 durch Drehen in eine Lagerposition sowie in eine Bereitstellungsposition zu überführen.

In dem von dem sockelartigen Anschlussabschnitt 5a des Applikators 5 umschlossenen Bereich ist zwischen dem Applikator und dem Behälter 1 ein Stopfen 9 vorgesehen, der die Kammer 4 verschließt. In der in Fig. 1 gezeigten Lagerposition des Stopfens 9 wird dieser verschiebefest in dem Behälter 1 gehalten. Dies wird durch mit einem ersten am Stopfen 9 angelegten Sperrelement 10 und einem zweiten, am Applikator 5 angelegten, Sperrelement 11 erreicht, welche in dieser Position derart zueinander ausgerichtet sind, dass diese ein Verschieben des Stopfens 9 relativ zu dem Applikator und damit auch relativ zu dem Behälter 1 verhindern. In dieser bevorzugten Ausführungsform weist der Stopfen 9 Kodierungsmittel 9a auf, die ein Drehen des Stopfens 9 relativ zu der Kammer 4 verhindern. Hierzu weist die Kammer 4 ebenfalls entsprechende Kodierungsmittel 4c auf. Dadurch wird eine richtige Positionierung des Stopfens 9 sichergestellt und ein unbeabsichtigtes Verdrehen der ersten und zweiten Sperrelemente 10 und 11 zueinander aufgrund einer Drehung des Stopfens 9 verhindert.

In dem in Fig. 1 gezeigten Zustand des Behälters 1 ist der Applikator 5 bereits so auf dem Behälter 1 vormontiert, dass der Behälter ohne weitere Montageschritte in Benutzung genommen werden kann. Dabei ist insbesondere von Vorteil, dass mit der erfindungsgemäßen Ausführungsform eine Metallkanüle 6 vom Hersteller vormontiert und verwendet werden kann, die auch bei der Aufbewahrung von korrosiven und/oder ätzenden Substanzen in der Kammer 4 bei längerer Lagerung (bspw. mehr als 2 Wochen) keine erkennbare Korrosion zeigt.

Das Gehäuse 2 des Behälters 1 ist auf der dem Applikator 5 abgewandten (proximalen) Seite vorzugsweise mit einem Halteabschnitt 2b versehen, der von dem Behälter 1 radial wegragt. Dabei weist der Halteabschnitt 2b einen im Vergleich zum Gehäuse 2 flanschartig vergrößerten Außendurchmesser auf, sodass der Behälter verschiebesicher in einer Ausbringpistole gehalten werden kann. Der Halteabschnitt 2b eignet sich somit zur Befestigung des Behälters in einer Ausbringpistole nach der US 8,602,775 B2. Eine Pistole 30 zur Aufnahme und Betätigung des Behälters ist in Fig. 12 dargestellt. Diese weist einen Betätigungsgriff 31, einen Haltebereich 32 sowie eine Aufnahmerinne 33 zum Einlegen des proximalen Endes eines Behälters 1 auf. Ein in Fig. 12 nicht gezeigter Stößel kann den Kolben 3 des Behälters 1 verschieben, um die Komponente aus der Kammer 4 auszubringen.

Die ersten Sperrelemente 10 des Stopfens 9 sind auch in der Fig. 2 gezeigt. Dabei bilden die Sperrelemente 10 ein auf der dem Applikator 5 zugewandten Seite des Stopfen 9 angeordnetes Kreuz, wobei die Sperrelemente 10 vorzugsweise radial durchgängig ausgeführt sind, also von dem einen radialen Ende des Stopfens 9 zu dem gegenüberliegenden radialen Ende reichen. Für die Ausführungsform der Fig. 1 und 2 mit einer einzigen Kammer 4 ist der Stopfen 9 vorzugsweise mit einem proximal herausragenden Verschlussabschnitt 12 ausgebildet. Durch diesen Verschlussabschnitt 12 wird die Kammer besonders sicher abgedichtet, sodass auch bei erhöhter thermischer Belastung (bspw. Temperaturen oberhalb 30 °C) und/oder mechanischen Erschütterungen des Behälters ein Herausfließen der in der Kammer 4 gelagerten Komponente an dem Stopfen 9 vorbei verhindert werden kann.

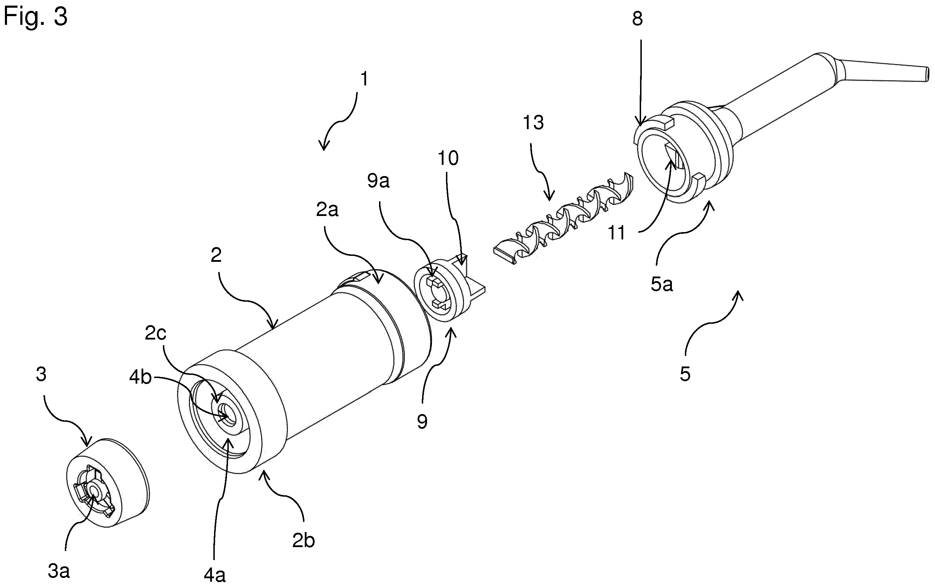

Die Fig. 3 bis 6 zeigen eine weitere Ausführungsform eines erfindungsgemäßen Behälters 1, der zwei Kammern 4a und 4b aufweist, welche in diesem Beispiel konzentrisch zueinander ausgeführt und durch eine Trennwand 2c voneinander getrennt sind. Das Volumen der Kammern 4a und 4b kann je nach gewünschten Verhältnis, bspw. 10:1 bis 1:1, insbesondere 1:1, 1,5:1, 2:1, 4:1, 5:1 und 10:1, der in den Kammern gelagerten Komponenten angepasst werden. In dieser Ausführungsform umfasst der Applikator 5 eine Mischwendel 13, welche frei drehbar in dem Applikator 5 gelagert sein kann. Alternativ ist die Mischwendel 13 einstückig mit dem Stopfen 9 verbunden.

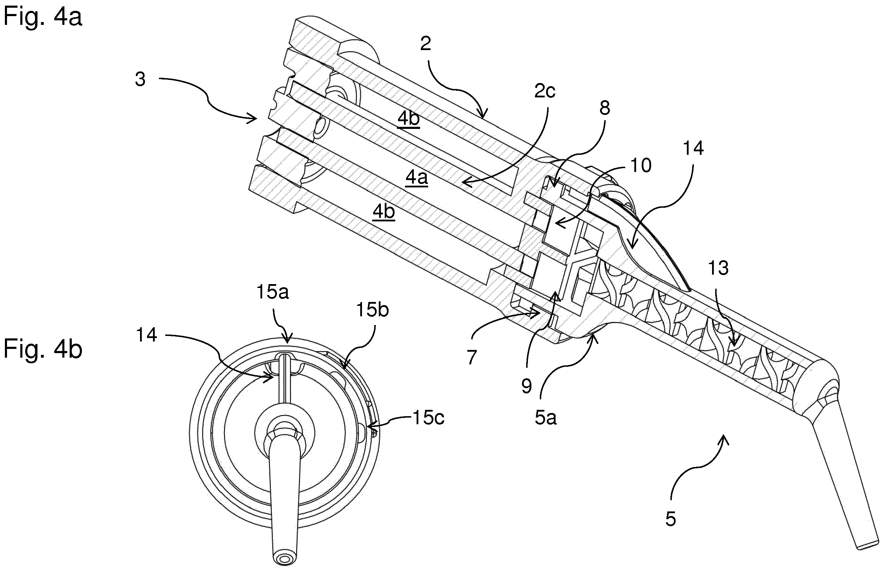

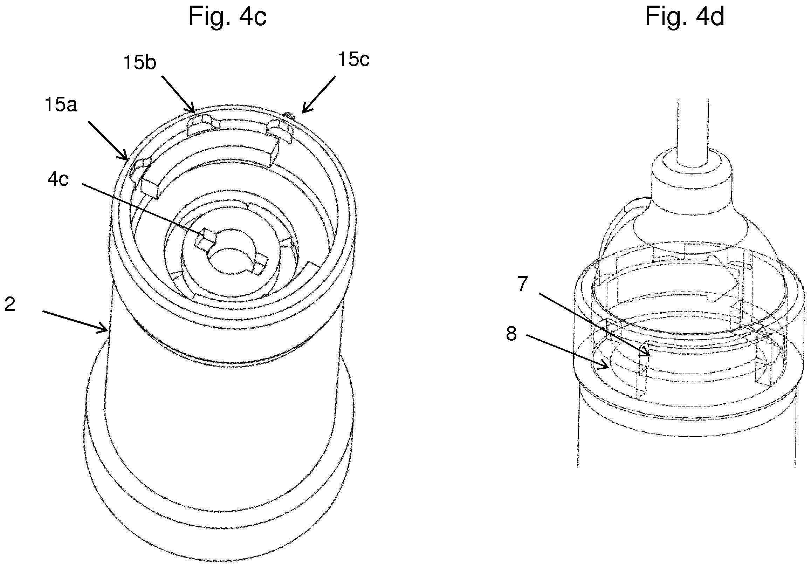

Die Fig. 4 bis 6 zeigen in Schnittansicht und Draufsicht unterschiedliche Positionen des Applikators 5 und deren Effekt auf die Sicherung des Stopfens 9 eines Behälters 1 nach Fig. 3.

Die Fig. 4a zeigt in Schnittansicht einen Behälter 1 in Montageposition gemäß der Fig. 3, wobei der Applikator 5 mit dem Gehäuse 2 lösbar verbunden ist. Weiterhin ist in der dargestellten Lage ein Rastmittel 14 dargestellt, welches eine Angriffsfläche zum händischen Verdrehen des Applikators 5 bildet. Die radial nach innen gerichteten Bajonettvorsprünge 7 des Behälters 1 und die radial nach außen gerichteten Bajonettvorsprünge 8 des Applikators 5 gleiten dazu in axialer Richtung aneinander vorbei, liegen also nicht übereinander. In dieser Position kann der Applikator 5 daher wieder zerstörungsfrei von dem Gehäuse 2 getrennt werden.

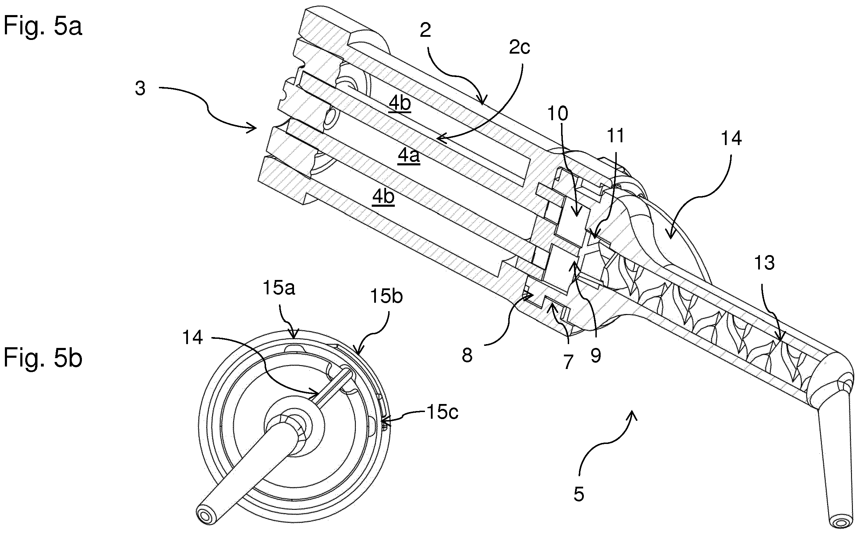

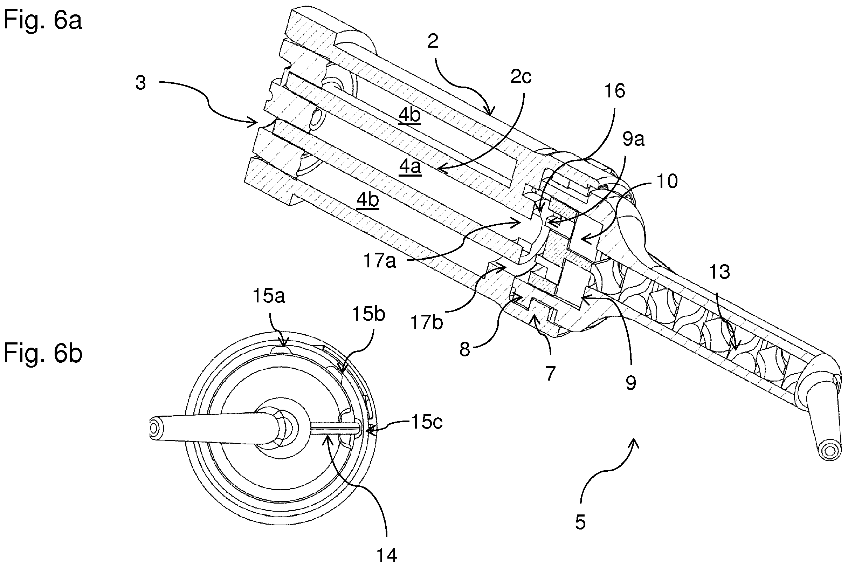

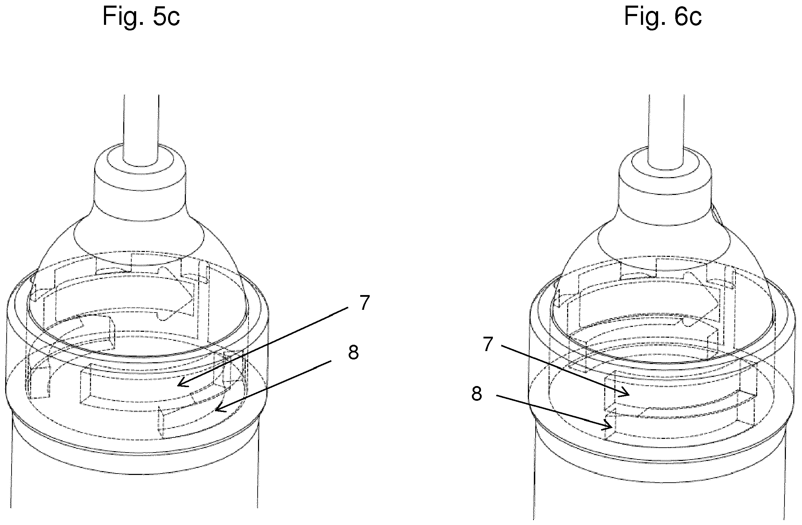

Die Fig. 4b zeigt als halbkreisförmige Aussparungen 15a, 15b und 15c ausgebildete Rastmittel des Behälters 1 an der dem Applikator zugewandten Seite des Gehäuses 2. In diese greift ein am Anschlussabschnitt 5a des Applikators 5 vorgesehenes Rastmittel 14 ein (Fig. 5b und 6b). Die Aussparungen 15a-c erlauben eine genaue und definierte Drehstellung des Applikators 5 in einer Montage- (Fig. 4), Lager- (Fig. 6) und Bereitstellungsposition (Fig. 6) und erschweren eine Drehung des Applikators 5, sodass ein unbeabsichtigtes Drehen verhindert wird. Die Fig. 4d, 5c und 6c zeigen die Stellung der radial nach innen gerichteten Bajonettvorsprünge 7 des Behälters 1 und der radial nach außen gerichteten Bajonettvorsprünge 8 des Applikators 5 in den drei Drehstellungen, d.h. in der Montageposition, in der Lagerposition und in der Bereitstellungsposition.

Die Form der am Behälter 1 ausgebildeten Rastmittel 15a-c (siehe Fig. 4c) ist derart, dass eine Drehung von der Montageposition in die Lagerposition über eine abgerundete Ecke der halbkreisförmigen Aussparung 15a erfolgt. Ebenso erfolgt die Drehung von der Lagerposition in die Bereitstellungsposition über deutlich abgerundete Ecken, sodass in dieser Drehrichtung wenig Kraft zur Drehung des Applikators aufgewendet werden muss.

Die Fig. 5a zeigt wiederum in Schnittansicht einen Behälter 1 nach der Fig. 3 in Lagerposition des Applikators 5. In der Lagerposition sind die ersten und zweiten Sperrelemente 10 und 11 derart zueinander ausgerichtet, dass ein Verschieben des Stopfens 9 in distaler Richtung verhindert wird. Außerdem liegen in der Lagerposition die Bajonettvorsprünge 7 und 8 zumindest teilweise übereinander (Fig. 5c), sodass eine Abnahme des Applikators 5 von dem Gehäuse 2 aus dieser Position nicht zerstörungsfrei möglich ist. Fig. 5b zeigt die Draufsicht des Behälter 1 in der Lagerposition nach Fig. 5a.

In der Fig. 6a ist eine Schnittansicht eines erfindungsgemäßen Behälters 1 nach der Ausführungsform der Fig. 3 in der Ausbringposition gezeigt. Zusätzlich ist zur Illustration der Funktionsweise der Strömungsverbindung zwischen den Kammern 4a/ 4b und dem Applikator 5 auch der Stopfen 9 aus seiner Lagerposition in seine Ausbringposition verschoben dargestellt, während der Kolben 3 noch in seiner Ausgangsstellung dargestellt ist. Die ersten und zweiten Sperrelemente 10 und 11 liegen nach dem Vorschieben des Stopfens 9 in axialer Richtung auf etwa gleicher Höhe sind aber seitlich zueinander versetzt.

In der Bereitstellungsposition der Fig. 6a und 6b liegen die Bajonettvorsprünge 7 und 8 derart übereinander, dass auch bei hohem Ausbringdruck der Applikator fest an dem Gehäuse 2 gehalten wird. Vorzugsweise ist der Formschluss der Bajonettvorsprünge 7 und 8 in der Bereitstellungsposition maximal, d.h. die Bajonettvorsprünge 7 und 8 liegen auf der gesamten Länge wenigstens eines der Bajonettvorsprünge übereinander.

In der Fig. 6a sind auch die Kodierungsmittel 9a des Stopfens 9 dargestellt. Schließt der Stopfen 9 die Kammern ab, wie in den Fig. 4a und 5a dargestellt, greifen diese Kodierungsmittel 9a in, hier an der Trennwand 2c vorgesehenen, Aussparungen 16 ein, wodurch nur eine bestimmte Ausrichtung des Stopfens 9 zum Verschließen der Kammern 4a, 4b möglich ist, da andernfalls die Kodierungsmittel 9a ein schlüssiges Aufsetzen des Stopfens 9 verhindern. Außerdem wird beim Verschieben des Stopfens 9, dieser in den Aussparungen 16 geführt, solange die Kodierungsmittel 9a noch in die Aussparungen 16 eingreifen. Dies verhindert ein Verkippen, Verkanten oder Verdrehen des Stopfens 9 beim Verschieben in die Ausbringposition, was bei herkömmlichen Behältern oftmals zu Problemen beim Austragen der Komponenten geführt hat.

Durch das Verschieben des Stopfens 9 in dessen Ausbringposition werden Ausbringöffnungen 17a und 17b der Kammern 4a bzw. 4b freigegeben. Die Komponenten können daher zunächst durch die Ausbringöffnungen 17a und 17b in den Freiraum zwischen Gehäuse und Stopfen strömen. Dabei kann es bereits zu einer gewissen Vormischung der Komponenten kommen, welche anschließend weiter durch den Stopfen 9 und an den Sperrelementen 10 und 11 vorbei in den Applikator 5 strömen, in welchem diese von der Mischwendel 13 weiter vermischt werden.

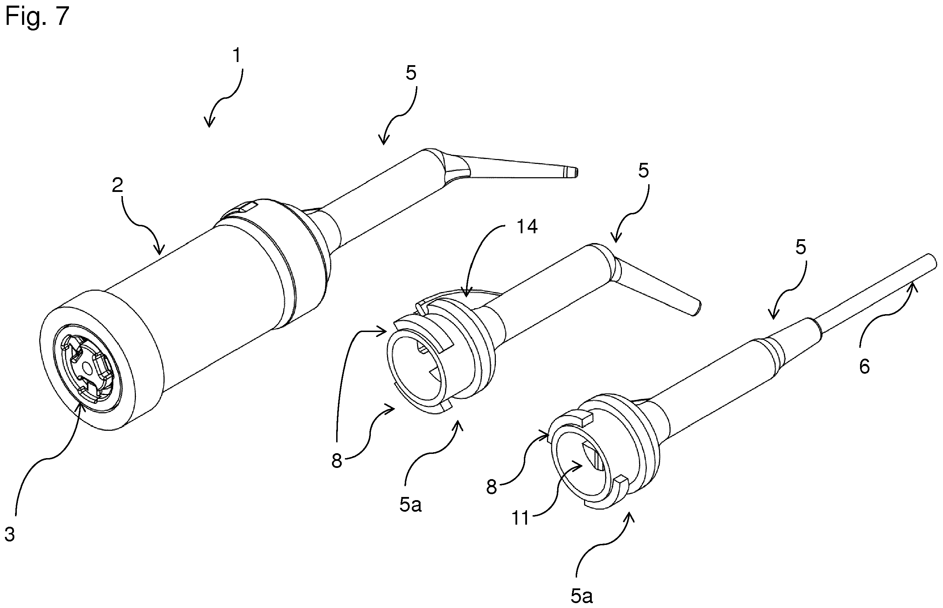

Die Fig. 7 zeigt einen Behälter 1 nach der zweiten Ausführungsform mit Applikator 5 sowie zwei weitere Beispiele geeigneter Applikatoren. Dabei sind auch das am Applikator 5 vorgesehene Rastmittel 14, die radial nach außen gerichteten Bajonettvorsprünge 8 sowie die im Inneren des Aufnahmeabschnitts 5a des Applikators 5 angeordneten zweiten Sperrelemente 11 dargestellt.

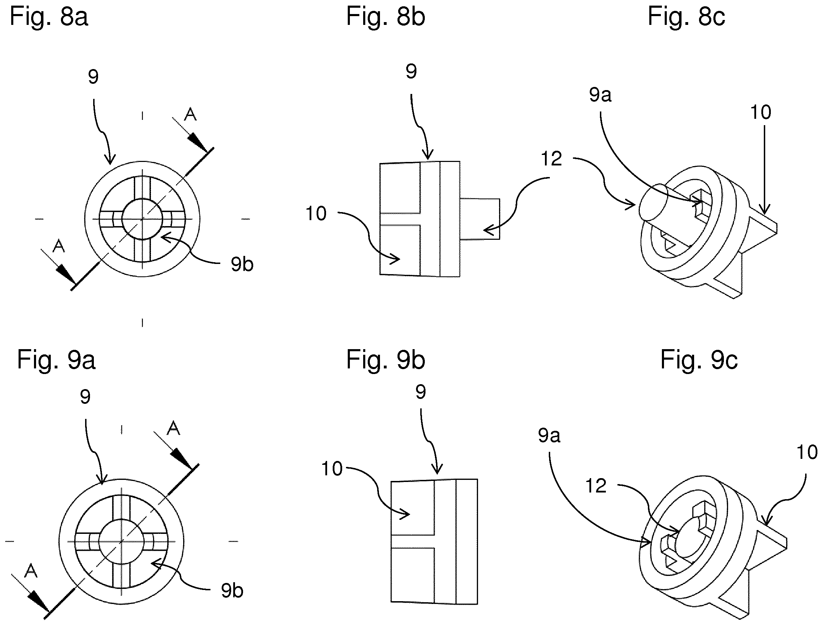

Die Fig. 8 zeigt den Stopfen 9 gemäß der ersten Ausführungsform mit Verschlussabschnitt 12, Kodierungsmittel 9a und Sperrelement 10. In der ebenfalls in Fig. 8 dargestellten Draufsicht auf den Stopfen 9 ist die vorzugsweise kreuzförmige Ausgestaltung des Sperrelements 10 gezeigt. Außerdem sind von dem Freiraum zwischen den Stegen des Sperrelements 10 gebildete Aussparungen 9b gezeigt, welche in der Ausbringposition des Stopfens einen Bypasskanal bilden.

Fig. 9 zeigt den Stopfen 9 für einen Behälter mit zwei konzentrischen Kammern. Dabei ist der Verschlussabschnitt 12 kürzer ausgeführt um ein höheres Volumen der innenliegenden Kammern zu ermöglichen.

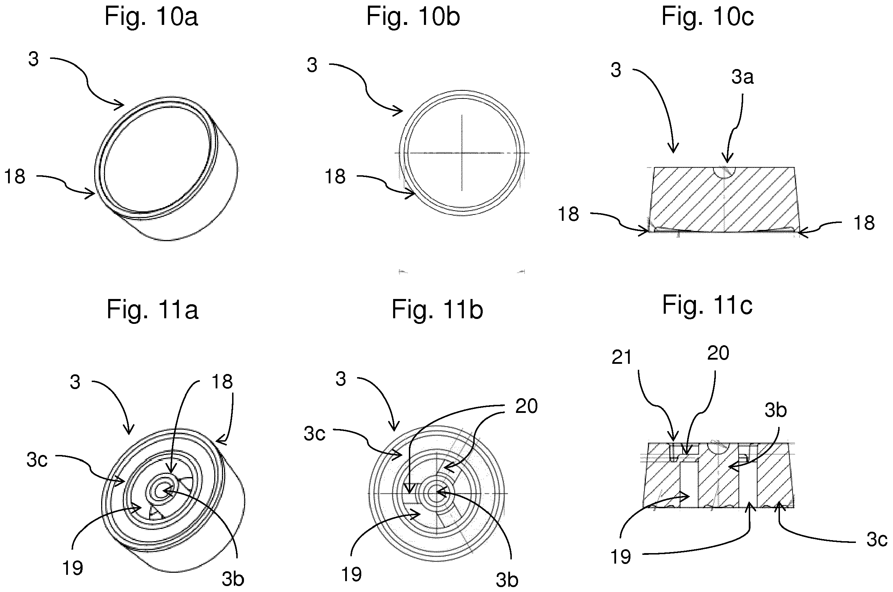

In Fig. 10a-c ist der Kolben 3 gemäß der ersten Ausführungsform mit Einkerbung 3a in einer perspektivischen Zeichnung (Fig. 10a), als Draufsicht (Fig. 10b) und Seitenansicht (Fig. 10c) gezeigt. Der Kolben 3 weist auf der dem Applikator 5 zugewandten (distalen) Seite eine Dichtung 18 auf, um die Kammer luftdicht abzuschließen. Dabei ist weiterhin von Vorteil, dass beim Austragen der Komponenten nahezu keine Rückstände innerhalb der Kammer verbleiben.

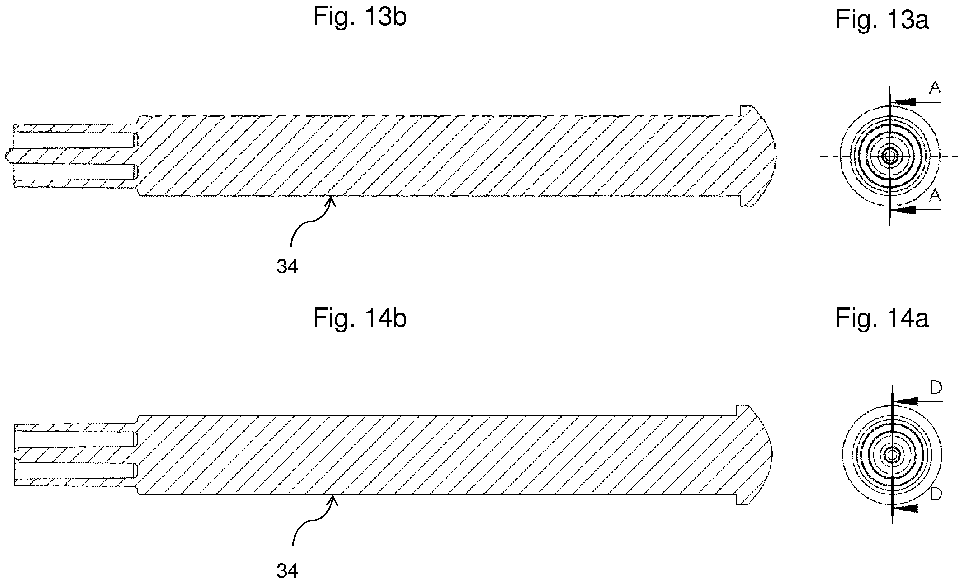

In den Fig. 13a, 13b, 14a und 14b werden die zur Einkerbung 3a korrespondierenden Kolbenstangen bzw. Stößel 34 und 34' gezeigt. Diese sind mit koaxialen Ausbringkonturen derart gestaltet, dass die innere Ausbringkontur gegenüber der äußeren Ausbringkontur hervorsteht (Fig. 13a, 13b) oder umgekehrt (Fig. 14a, 14b). Auf diese Weise kann ein potentielles Vorlaufen einer Basis- oder Katalysatorkomponente in einer der Kammern des Behälters kompensiert werden. Diese Stößel können auch für Behälter mit nur einer Kammer eingesetzt werden, wobei hier die Variante nach Fig. 14a und 14b bevorzugt wird, da durch die äußere ringförmige Auflagefläche des Stößels die Ausbringkraft besser auf den Kolben übertragen werden kann.

Zusätzlich kann diese Ausbringkontur auch für eine 1-Komponenten-Compule verwendet werden. Besonders bevorzugt steht hier die äußere Ausbringkontur über die innere Ausbringkontur hervor, um hier die Kräfte über einer größeren Fläche gleichmäßig zu verteilen.

In Fig. 11a-c ist der Kolben 3 für einen Behälter mit zwei konzentrisch ausgebildeten Kammern gezeigt. In der in Fig. 11a dargestellten perspektivischen Zeichnung ist eine ringförmige, axial nahezu durchgängige Aussparung 19 gezeigt, welche beim Eindrücken des Kolben in das Gehäuse 2 mit den Kammern 4a, 4b die Trennwand 2c aufnimmt. Der Kolben 3 weist den zwei Kammern 4a, 4b entsprechende Abschnitte 3b (zylindrisch) und 3c (ringförmig) auf, welche mit Dichtungen 18 an dem Übergang einer Kammer zu einer die Kammer begrenzende Wand, bspw. die Trennwand 2c, angeordnet sind. Obwohl der radial innere Abschnitt 3b und der radial äußere Abschnitt 3c in den Kammern separat geführt wird, ist der Kolben 3 einer bevorzugten Ausgestaltung einstückig ausgebildet, was die Montage des Behälters und die Befüllung mit den Komponenten deutlich vereinfacht. Dazu sind zwischen den Abschnitten 3b und 3c, vorzugsweise drei, Stege 20 vorgesehen, welche beim Einführen des Kolbens 3 in den Behälter durch die Trennwand 2c abgebrochen werden, sodass die Abschnitte 3b und 3c separat in den Kammern 4a und 4b geführt werden können. Mit anderen Worten teilt sich der einstückig ausgeführte Kolben 3 bei der Montage automatisch in zwei separat in den Kammern geführte Abschnitte 3b und 3c auf.

Dabei ist es bevorzugt, wenn die Stege 20 an dem radial inneren Abschnitt 3b mit einer Abbruchkante festgemacht sind, welche im Vergleich zu der Dicke der Stege 20 deutlich dünner ausgeführt ist und dadurch eine Sollbruchstelle bildet. Weiter wird es bevorzugt, die Stege 20 an dem radial äußeren Abschnitt 3c derart ausbilden, dass ein Abbrechen der Stege 20 an dem Abschnitt 3c verhindert wird. Mit anderen Worten brechen die Stege 20 nur an dem radial inneren Abschnitt 3b ab und bleiben mit dem radial äußeren Abschnitt 3c verbunden. Dadurch kann verhindert werden, dass die Stege 20 vollständig von den Abschnitten 3b und 3c getrennt werden und diese beim Hineindrücken des Kolbens 3 in das Gehäuse 2 denselben blockieren könnten.

In einer weiteren bevorzugten Ausführung weist der radial äußere Abschnitt 3c eine Aussparung 21 auf, welche die am radial inneren Abschnitt 3b abgebrochenen Stege 20 aufnehmen können. Mit anderen Worten klappen die Stege 20 bei Einführen des Kolbens 3 in das Gehäuse 2 in die dafür vorgesehenen Aussparungen 21 des radial äußeren Abschnitts 3c weg und liegen anschließend im Wesentlichen bündig an der der Trennwand 2c zugewandten Seite des Abschnitts 3c an. Die Aussparungen 21 erlauben daher eine Aufnahme der Stege 20 womit ein Blockieren der Abschnitte 3b und 3c verhindert wird. Ein weiterer Effekt der Aussparungen 21 ist darin zu sehen, dass eine Beschädigung der Trennwand 2c durch die am Abschnitt 3b abgebrochenen Stege 20 verhindert wird wodurch eine Undichtigkeit einer Kammer aufgrund einer Beschädigung der Wände und eine damit deutlich verschlechterte Lagerstabilität verhindert wird.

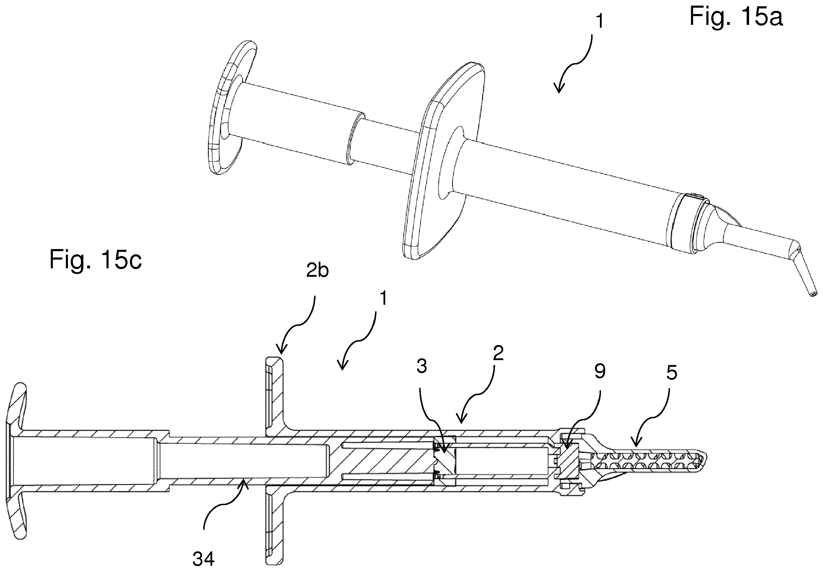

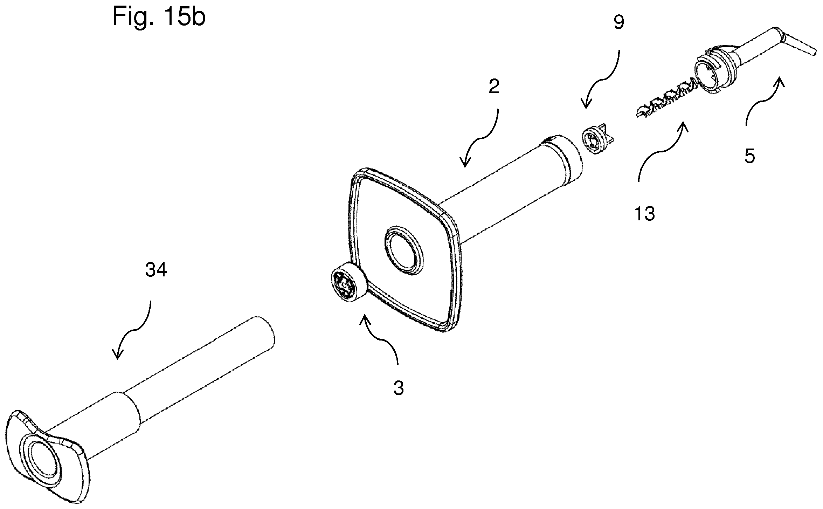

In den Fig. 15a bis 15c ist eine weitere Ausführungsform der Erfindung dargestellt. Der Behälter 1 ist dabei als eine Spritze ausgestaltet, die ohne eine Pistole (Fig. 12) manuell bedienbar ist. Hierzu ist der Halteabschnitt 2b gegenüber den Ausführungsformen nach den Fig. 1 oder 3 vergrößert als ein Flansch ausgebildet, der zur Abstützung von Fingern bei der Betätigung dient. Zusätzlich ist ein Stößel 34 vorgesehen, an dessen proximalem Ende ebenfalls eine vergrößerte Abstützfläche für die manuelle Betätigung vorgesehen ist. In der dargestellten Ausführungsform ist der Behälter mit zwei zueinander koaxialen Kammern und mit einem Stößel 34 mit einem stiftartigen inneren Bereich und einem zylindrischen äußeren Bereich ausgestaltet, d.h. für zwei Komponenten. Entsprechend weist auch der Kolben 3 zwei getrennte Kolbenelemente auf. Es ist jedoch auch möglich, die spritzenartige Ausgestaltung für Behälter mit nur einer Kammer oder mit mehreren Kammern vorzusehen.

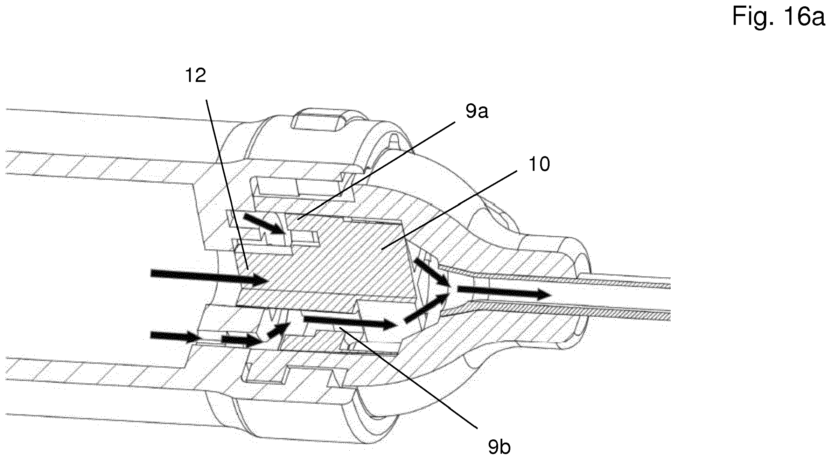

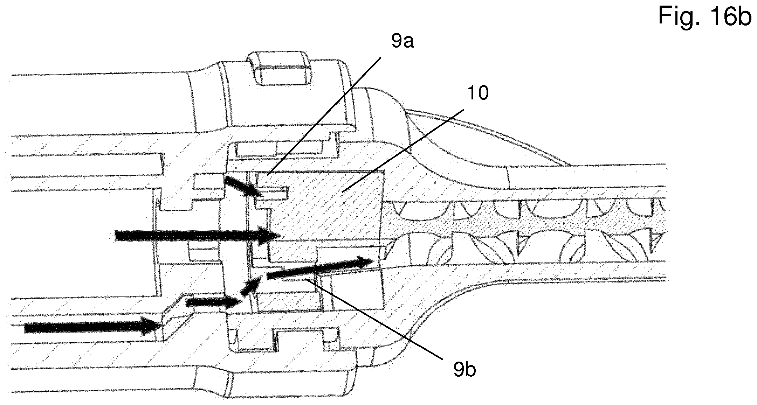

Der Strömungsweg der Komponenten im geöffneten Zustand des Behälters aus der jeweiligen Kammer in den Applikator ist in den Fig. 16a und 16b für einen Ein-Komponenten-Behälter und einen Zwei-Komponenten-Behälter dargestellt. Hieraus ist ersichtlich, wie die in den Fig. 16a bzw. 16b durch Pfeile dargestellten Komponenten auf den jeweiligen Stopfen 9 Druck ausüben, um diesen in Richtung zu dem Applikator 5 zu verschieben, bis die Komponenten dann durch den jeweiligen Bypasskanal 9b durch den Stopfen 9 bzw. an diesem vorbei strömen können.

**Bezugszeichen**

| | | | |
|---|---|---|---|
| 1 | Behälter | 9b | Bypasskanal |
| 2 | Gehäuse | 10 | Sperrelement |
| 2a | Aufnahme | 11 | Sperrelement |
| 2b | Halteabschnitt | 12 | Verschlussabschnitt |
| 2c | Trennwand | 13 | Mischwendel |
| 3 | Kolben | 14 | Rastmittel |
| 3a | Einkerbung | 15a - 15c | Aussparung |
| 3b | zylindrischer Abschnitt | 16 | Aussparung |
| 3c | ringförmiger Abschnitt | 17a, 17b | Ausbringöffnung |
| 4 | Kammer | 18 | Dichtung |
| 4a, 4b | Kammer | 19 | Aussparung |
| 4c | Kodiermittel | 20 | Steg |
| 5 | Applikator | 21 | Aussparung |
| 5a | Anschlussabschnitt | | |
| 6 | Metallkanüle | 30 | Pistole |
| 7 | Bajonettvorsprünge | 31 | Betätigungsgriff |
| 8 | Bajonettvorsprünge | 32 | Haltebereich |
| 9 | Stopfen | 33 | Aufnahmerinne |
| 9a | Kodiermittel | 34 | Stößel |

## Patentansprüche

1. Behälter (1) zum Aufbewahren und Ausbringen wenigstens einer Komponente mit wenigstens einer Kammer (4, 4a, 4b), die auf einer ersten Seite von einem Stopfen (9) verschlossen ist, und mit einem auf der ersten Seite befestigten Applikator (5), wobei der Stopfen (9) relativ zu der Kammer (4, 4a, 4b) aus einer Lagerposition, in der eine Strömungsverbindung zwischen der Kammer (4, 4a, 4b) und dem Applikator (5) durch den Stopfen (9) unterbrochen ist, in eine Ausbringposition, in der die Kammer (4, 4a, 4b) mit dem Applikator (5) in Strömungsverbindung steht, verschiebbar ist, **dadurch gekennzeichnet, dass** eine Strömungsverbindung zwischen der wenigstens einen Kammer (4, 4a, 4b) und dem der wenigstens einen Kammer (4, 4a, 4b) abgewandten Seite des Applikators (5) freigegeben wird durch eine relative Drehbewegung des Applikators (5) zu dem Behälter (1) und eine anschließende axiale Relativbewegung des Stopfens (9) zu dem Behälter (1) und dem Applikator (5), und dass der Stopfen (9) ein erstes Sperrelement (10) und der Applikator (5) ein zweites Sperrelement (11) aufweist, und dass der Applikator (5) relativ zu der Kammer aus einer Lagerposition, in der die Sperrelemente (10, 11) so zueinander ausgerichtet sind, dass diese ein Verschieben des Stopfens (9) verhindern, in eine Bereitstellungsposition drehbar ist, in der die Sperrelemente (10, 11) so zueinander ausgerichtet sind, dass diese ein axiales Verschieben des Stopfens (9) ermöglichen.

2. Behälter nach Anspruch 1, **dadurch gekennzeichnet, dass** an dem Behälter (1) und/oder dem Applikator (5) Rastmittel (14, 15a, 15b, 15c) vorgesehen sind, die eine relative Drehbewegung des Applikators (5) zu dem Behälter (1) in einer ersten Drehrichtung erlauben und eine Drehbewegung in einer anderen Drehrichtung verhindern.

3. Behälter nach Anspruch 1 oder 2, **dadurch gekennzeichnet, dass** der Applikator (5) in der Lagerposition und in der Bereitstellungsposition jeweils axial an dem Behälter (1) festgelegt ist.

4. Behälter nach einem der Ansprüche 1 bis 3, **dadurch gekennzeichnet, dass** der Applikator (5) aus einer Montageposition, in der der Applikator (5) axial auf den Behälter (1) aufsteckbar ist, relativ zu der Kammer (4, 4a, 4b) zunächst in die Lagerposition und dann weiter in die Bereitstellungsposition drehbar ist.

5. Behälter nach einem der vorhergehenden Ansprüche, **dadurch gekennzeichnet, dass** der Behälter (1) eine Aufnahme (2a) für den Applikator (5) mit wenigstens einem radial nach innen gerichteten Bajonettvorsprung (7) aufweist und der Applikator (5) einen in die Aufnahme (2a) einsteckbaren Anschlussabschnitt (5a) mit wenigstens einem radial nach außen gerichteten Bajonettvorsprung (8) aufweist und auf seinem der Aufnahme (2a) gegenüberliegenden Ende einen flanschartigen Halteabschnitt (2b) mit vergrößertem Außendurchmesser aufweist.

6. Behälter nach einem der Ansprüche 2 bis 5, **dadurch gekennzeichnet, dass** die Rastmittel (14, 15a, 15b, 15c) eine relative Drehbewegung zwischen Applikator (5) und Behälter (1) erschweren.

7. Behälter nach einem der Ansprüche 1 bis 6, **dadurch gekennzeichnet, dass** das erste Sperrelement (10) ein Steg oder ein Vorsprung am Applikator (5) ist, und dass das zweite Sperrelement (11) ein Steg oder ein Vorsprung ist, der zumindest in der Lagerposition den Steg oder Vorsprung des Stopfens (9) überdeckt.

8. Behälter nach einem der vorhergehenden Ansprüche, **dadurch gekennzeichnet, dass** der Stopfen (9) wenigstens einen Bypasskanal (9b) aufweist, durch den in seiner Ausbringposition eine Strömungsverbindung von der wenigstens einen Kammer (4, 4a, 4b) zu dem Applikator (5) hergestellt wird.

9. Behälter nach einem der Ansprüche 2 bis 8, **dadurch gekennzeichnet, dass** die wenigstens eine Kammer (4, 4a, 4b) wenigstens eine Auslassöffnung (17a, 17b) aufweist, und dass der Stopfen (9) wenigstens einen Verschlussabschnitt (12) aufweist, der in der Lagerposition des Stopfens (9) die Auslassöffnung (17a, 17b) verschließend in diese eingesteckt ist.

10. Behälter nach einem der Ansprüche 2 bis 9, **dadurch gekennzeichnet, dass** der Stopfen (9) eine vorzugsweise kreisrunde Außenkontur aufweist und sowohl in der Lagerposition als auch in der Ausbringposition drehfest in dem Behälter (1) geführt ist.

11. Behälter nach einem der vorhergehenden Ansprüche, **dadurch gekennzeichnet, dass** der Stopfen (9) eine im Wesentlichen kreisscheibenförmige Grundform hat und auf seiner der wenigstens einen Kammer (4, 4a, 4b) zugewandten Seite und/oder an seinem Außenumfang Dichtmittel aufweist, auf seiner der wenigstens einen Kammer (4, 4a, 4b) abgewandten Seite ein durch zwei zueinander rechtwinklig verlaufende Stege gebildetes Sperrelement (10) aufweist und wenigstens einen Durchbruch als Bypasskanal (9b) aufweist.

12. Behälter nach einem der vorhergehenden Ansprüche, **dadurch gekennzeichnet, dass** die wenigstens eine Kammer (4, 4a, 4b) auf ihrer dem Stopfen (9) abgewandten Seite mit einem Kolben (3) verschlossen ist, der über wenigstens eine Sollbruchstelle einstückig mit dem Behälter (1) verbunden ist.

13. Behälter nach einem der vorhergehenden Ansprüche mit einem separaten Stößel (34), der zum Einstecken in die wenigstens eine Kammer (4, 4a, 4b) ausgebildet ist.

14. Verfahren zum Ausbringen wenigstens einer Komponente aus einem Behälter (1) mit wenigstens einer Kammer (4, 4a, 4b), die auf einer ersten Seite von einem Stopfen (9) verschlossen ist, und mit einem auf der ersten Seite drehbar befestigten Applikator (5), der in einer Lagerposition eine axiale Bewegung des Stopfens (9) verhindert, wobei der Stopfen (9) ein erstes Sperrelement (10) und der Applikator (5) ein zweites Sperrelement (11) aufweist, wobei das Verfahren folgende Schritte aufweist:
i) Drehen des Applikators (5) relativ zu dem Behälter (1) aus einer Lagerposition, in der die Sperrelemente (10, 11) so zueinander ausgerichtet sind, dass diese ein Verschieben des Stopfens (9) verhindern, in eine Bereitstellungsposition, in der der Applikator (5) eine axiale Bewegung des Stopfens (9) nicht verhindert, wobei in der Bereitstellungsposition die Sperrelemente (10, 11) so zueinander ausgerichtet sind, dass diese ein axiales Verschieben des Stopfens (9) ermöglichen,
ii) Verschieben des Stopfens (9) aus einer Lagerposition, in der eine Strömungsverbindung zwischen der Kammer (4, 4a, 4b) und dem Applikator (5) durch den Stopfen (9) unterbrochen ist, in eine Ausbringposition, in der die Kammer (4, 4a, 4b) mit dem Applikator (5) in Strömungsverbindung steht.

15. Verfahren nach Anspruch 14, **dadurch gekennzeichnet, dass** Drehen des Applikators (5) durch an dem Behälter (1) und/oder dem Applikator (5) vorgesehene Rastmittel (14, 15a, 15b, 15c) in einer ersten Drehrichtung erlaubt und Drehen in einer anderen Drehrichtung verhindert wird.

## Claims

1. A container (1) for storing and dispensing at least one component with at least one chamber (4, 4a, 4b), which is closed on a first side by a plug (9), and with an applicator (5) fastened on the first side, wherein the plug (9) can be displaced relative to the chamber (4, 4a, 4b) out of a storage position, in which a flow connection between the chamber (4, 4a, 4b) and the applicator (5) is interrupted by the plug (9), into a dispensing position, in which the chamber (4, 4a, 4b) is in a flow connection with the applicator (5), **characterised in that** a flow connection between the at least one chamber (4, 4a, 4b) and the side of the applicator (5) facing away from the at least one chamber (4, 4a, 4b) is opened by a relative rotational movement of the applicator (5) with respect to the container (1) and a subsequent axial relative movement of the plug (9) with respect to the container (1) and the applicator (5), and that the plug (9) comprises a first stop element (10) and the applicator (5) a second stop element (11), and that the applicator (5) can be rotated relative to the chamber out of the storage position, in which the stop elements (10, 11) are aligned with respect to one another in such a way that they prevent a displacement of the plug (9), into a readiness position, in which the stop elements (10, 11) are aligned with respect to one another in such a way that they enable an axial displacement of the plug (9).

2. The container according to claim 1, **characterised in that** catch means (14, 15a, 15b, 15c) are provided on the container (1) and/or the applicator (5), which catch means permit a relative rotational movement of the applicator (5) relative to the container (1) in the first direction of rotation and prevent a rotational movement in another direction of rotation.

3. The container according to claim 1 or 2, **characterised in that** the applicator (5) in the storage position and in the readiness position is in each case fixed axially to the container (1).

4. The container according to any one of claims 1 to 3, **characterised in that** the applicator (5) can be rotated out of an assembly position, in which the applicator (5) can be inserted axially onto the container (1), relative to the chamber (4, 4a, 4b) first into the storage position and then onward into the readiness position.

5. The container according to any one of the preceding claims, **characterised in that** the container (1) comprises a holding fixture (2a) for the applicator (5) with at least one radially inwardly directed bayonet projection (7) and the applicator (5) comprises a connection portion (5a) with at least one radially outwardly directed bayonet projection (8) which can be inserted into the holding fixture (2a) and comprises on its end lying opposite the holding fixture (2a) a flange-like holding portion (2b) with an enlarged outer diameter.

6. The container according to any one of claims 2 to 5, **characterised in that** the catch means (14, 15a, 15b, 15c) make a relative rotational movement between the applicator (5) and the container (1) difficult.

7. The container according to any one of claims 1 to 6, **characterised in that** the first stop element (10) is a web or a projection on the applicator (5), and that the second stop element (11) is a web or a projection which, at least in the storage position, overlaps the web or projection of the plug (9).

8. The container according to any one of the preceding claims, **characterised in that** the plug (9) comprises at least one bypass channel (9b) through which, in its dispensing position, a flow connection is produced from the at least one chamber (4, 4a, 4b) to the applicator (5).

9. The container according to any one of claims 2 to 8, **characterised in that** the at least one chamber (4, 4a, 4b) comprises at least one outlet opening (17a, 17b), and that the plug (9) comprises at least one closure section (12) which, in the storage position of the plug (9), is plugged in a sealing manner into the outlet opening (17a, 17b).

10. The container according to any one of claims 2 to 9, **characterised in that** the plug (9) has a preferably circular outer contour and is introduced non-rotatably into the container (1) when it is both in the storage position and in the dispensing position.

11. The container according to any one of the preceding claims, **characterised in that** the plug (9) has an essentially circular disc-shaped basic form and comprises sealing means on its side facing the at least one chamber (4, 4a, 4b) and/or at its outer circumference, comprises a stop element (10) formed by two webs running at right angles to one another on its side facing away from the at least one chamber (4, 4a, 4b) and comprises at least one perforation as a bypass channel (9b).

12. The container according to any one of the preceding claims, **characterised in that** the at least one chamber (4, 4a, 4b) is closed with a plunger (3) on its side facing away from the plug (9), which plunger is connected by at least one predetermined breaking point in one piece with the container (1).

13. The container according to any one of the preceding claims with a separate ram (34), which is constituted for insertion into at least one chamber (4, 4a, 4b).

14. A method for dispensing at least one component from a container (1) with at least one chamber (4, 4a, 4b), which is closed on a first side by a plug (9), and with an applicator (5), which is fastened rotatably on the first side and which, in a storage position, prevents an axial movement of the plug (9), wherein the plug (9) comprises a first stop element (10) and the applicator (5) comprises a second stop element (11), wherein the method comprises the following steps:
i) Rotation of the applicator (5) relative to the container (1) out of a storage position, in which the stop elements (10, 11) are aligned with one another in such a way that they prevent a displacement of the plug (9), into a readiness position, in which the applicator (5) does not prevent the axial movement of the plug (9), wherein in the readiness position the stop elements (10, 11) are aligned with one another in such a way that they enable an axial displacement of the plug (9),
ii) Displacement of the plug (9) out of the storage position, in which a flow connection between the chamber (4, 4a, 4b) and the applicator (5) is interrupted by the plug (9), into a dispensing position, in which the chamber (4, 4a, 4b) is in a flow connection with the applicator (5).

15. The method according to claim 14, **characterised in that** rotation of the applicator (5) is allowed in a first direction of rotation by catch means (14, 15a, 15b, 15c) provided on the container (1) and/or the applicator (5) and rotation in another direction of rotation is prevented.

## Revendications

1. Récipient (1) destiné à conserver et à distribuer au moins un composant, disposant d'au moins un compartiment (4, 4a, 4b), qui sur un premier côté est fermé par un bouchon (9) et disposant d'un applicateur (5) fixé sur le premier côté, le bouchon (9) étant déplaçable par rapport au compartiment (4, 4a, 4b) à partir d'une position de stockage, dans laquelle une liaison d'écoulement entre le compartiment (4, 4a, 4b) et l'applicateur (5) est interrompue par le bouchon (9) dans une position de distribution, dans laquelle le compartiment (4, 4a, 4b) est en liaison d'écoulement avec l'applicateur (5), **caractérisé en ce qu'**une liaison d'écoulement entre l'au moins un compartiment (4, 4a, 4b) et le côté de l'applicateur (5) opposé à l'au moins un compartiment (4, 4a, 4b) est libérée par un déplacement en rotation de l'applicateur (5) par rapport au récipient (1) et un déplacement axial relatif consécutif du bouchon (9) par rapport au récipient (1) et à l'applicateur (5), et **en ce que** le bouchon (9) comporte un premier élément de blocage (10) et **en ce que** l'applicateur (5) comporte un deuxième élément de blocage (11) et **en ce que** l'applicateur (5) est rotatif par rapport au compartiment, d'une position de stockage, dans laquelle les éléments de blocage (10, 11) sont orientés l'un par rapport à l'autre de sorte à empêcher un déplacement du bouchon (9) dans une position de mise à disposition dans laquelle les éléments de blocage (10, 11) sont orientés l'un par rapport à l'autre, de telle sorte que ces derniers permettent un déplacement axial du bouchon (9).

2. Récipient selon la revendication 1, caractérisé en ce sur le récipient (1) et/ou sur l'applicateur (5) sont prévus des moyens d'enclenchement (14, 15a, 15b, 15c) qui permettent un déplacement en rotation de l'applicateur (5) relativement au récipient (1) dans un premier sens de rotation et empêchent un déplacement en rotation dans un autre sens de rotation.

3. Récipient selon la revendication 1 ou 2, **caractérisé en ce que** dans la position de stockage et dans la position de mise à disposition, l'applicateur (5) est chaque fois immobilisé en direction axiale sur le récipient (1).

4. Récipient selon l'une quelconque des revendications 1 à 3, **caractérisé en ce que** l'applicateur (5) est rotatif d'une position de montage, dans laquelle l'applicateur (5) est emboîtable en direction axiale sur le récipient (1), par rapport au compartiment (4, 4a, 4b), d'abord dans la position de stockage et ensuite, ultérieurement, dans la position de mise à disposition.

5. Récipient selon l'une quelconque des revendications précédentes, **caractérisé en ce que** le récipient (1) comporte un logement (2a) pour l'applicateur (5), pourvu d'au moins une fermeture à baïonnette (7) orientée vers l'intérieur en direction radiale et **en ce que** l'applicateur (5) comporte un tronçon de raccordement (5a) enfichable dans le logement (2a), pourvu d'au moins un fermeture à baïonnette (8) orientée vers l'extérieur en direction radiale et sur son extrémité opposée au logement (2a), comporte un segment de retenue (2b) sous la forme d'une bride, avec un diamètre extérieur agrandi.

6. Récipient selon l'une quelconque des revendications 2 à 5, **caractérisé en ce que** les moyens d'enclenchement (14, 15a, 15b, 15c) compliquent un déplacement en rotation relatif entre l'applicateur (5) et le récipient (1).

7. Récipient selon l'une quelconque des revendications 1 à 6, **caractérisé en ce que** le premier élément de blocage (10) est une barrette ou une saillie sur l'applicateur (5) et **en ce que** le deuxième élément de blocage (11) est une barrette ou une saillie, qui au moins dans la position de stockage, recouvre la barrette ou la saillie du bouchon (9).

8. Récipient selon l'une quelconque des revendications précédentes, **caractérisé en ce que** le bouchon (9) comporte au moins un canal de dérivation (9b) à travers lequel, dans sa position de distribution, une liaison d'écoulement est établie à partir de l'au moins un compartiment (4, 4a, 4b) vers l'applicateur (5) .

9. Récipient selon l'une quelconque des revendications 2 à 8, **caractérisé en ce que** l'au moins compartiment (4, 4a, 4b) comporte au moins un orifice de sortie (17a, 17b) et **en ce que** le bouchon (9) comporte au moins un tronçon de fermeture (12), qui dans la position de stockage du bouchon (9) est inséré dans l'orifice de sortie (17a, 17b) en fermant celui-ci.

10. Récipient selon l'une quelconque des revendications 2 à 9, **caractérisé en ce que** le bouchon (9) présente un contour extérieur de préférence de forme circulaire et aussi bien dans la position de stockage, qu'également dans la position de distribution est guidé de manière solidaire en rotation dans le récipient (1).

11. Récipient selon l'une quelconque des revendications précédentes, **caractérisé en ce que** le bouchon (9) présente une forme de base sensiblement en forme de disque circulaire et sur son côté dirigé vers l'au moins un compartiment (4, 4a, 4b) et/ou sur son pourtour extérieur, comporte des moyens d'étanchéité, sur son côté opposé à l'au moins un compartiment (4, 4a, 4b), comporte un élément de blocage (10) formé de deux barrettes s'écoulant à angle droit l'une par rapport à l'autre et comporte au moins un ajour, en tant que canal de dérivation (9b).

12. Récipient selon l'une quelconque des revendications précédentes, **caractérisé en ce que** sur son côté opposé au bouchon (9), l'au moins un compartiment (4, 4a, 4b) est fermé par un piston (3), qui au moins par l'intermédiaire d'un point de rupture théorique, est assemblé en monobloc avec le récipient (1).

13. Récipient selon l'une quelconque des revendications précédentes, pourvu d'un coulisseau (34) séparé, qui est conçu pour être inséré dans l'au moins un compartiment (4, 4a, 4b).

14. Procédé, destiné à distribuer au moins un composant à partir d'un récipient (1) pourvu d'au moins un compartiment (4, 4a, 4b), qui sur un côté est fermé par un bouchon (9), et pourvu d'un applicateur (5) fixé de manière rotative sur le premier côté, qui dans une position de stockage empêche un déplacement axial du bouchon (9), le bouchon (9) comportant un premier élément de blocage (10) et l'applicateur (5) comportant un deuxième élément de blocage (11), le procédé comportant les étapes suivantes, consistant à
i) faire tourner l'applicateur (5) par rapport au récipient (1) d'une position de stockage, dans laquelle les éléments de blocage (10, 11) sont orientés l'un par rapport de telle sorte qu'il empêchent un déplacement du bouchon (9) dans une position de mise à disposition, dans laquelle l'applicateur (5) n'empêche pas un déplacement axial du bouchon (9), dans la position de mise à disposition, les éléments de blocage (10, 11) étant orientés l'un par rapport à l'autre de sorte à permettre un déplacement axial du bouchon (9),
ii) déplacer le bouchon (9) d'une position de stockage, dans laquelle une liaison d'écoulement entre le compartiment (4, 4a, 4b) et l'applicateur (5) est interrompue par le bouchon (9) dans une position de distribution, dans laquelle le compartiment (4, 4a, 4b) est en liaison d'écoulement avec l'applicateur (5).

15. Procédé selon la revendication 14, **caractérisé en ce qu'**une rotation de l'applicateur (5) est permise dans un premier sens de rotation et est empêchée dans un autre sens de rotation par des moyens d'enclenchement (14, 15a, 15b, 15c) prévus sur le récipient (1) et/ou sur l'applicateur (5).
